(19) Europäisches Patentamt
European Patent Office
Office européen des brevets

(11) **EP 3 173 064 B1**

(12) **EUROPEAN PATENT SPECIFICATION**

(45) Date of publication and mention
of the grant of the patent:
**12.09.2018 Bulletin 2018/37**

(51) Int Cl.:
*A61K 8/891* *(2006.01)*      *A61K 8/31* *(2006.01)*
*A61K 8/37* *(2006.01)*      *A61K 8/42* *(2006.01)*
*A61K 8/34* *(2006.01)*      *A61Q 19/00* *(2006.01)*

(21) Application number: **16200620.9**

(22) Date of filing: **25.11.2016**

(54) **OIL-IN-WATER EMULSION GEL COMPOSITION, EXTERNAL PREPARATION FOR SKIN AND PRODUCING METHOD OF OIL-IN-WATER EMULSION COMPOSITION**

ÖL-IN-WASSER-EMULSION-GELZUSAMMENSETZUNG, EXTERNES PRÄPARAT FÜR HAUT UND VERFAHREN ZUR HERSTELLUNG DER ÖL-IN-WASSER-EMULSIONSZUSAMMENSETZUNG

COMPOSITION DE GEL D'ÉMULSION HUILE DANS EAU, PRÉPARATION EXTERNE POUR LA PEAU ET PROCÉDÉ DE PRODUCTION DE COMPOSITION D'ÉMULSION HUILE-DANS-EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2015 JP 2015233601**

(43) Date of publication of application:
**31.05.2017 Bulletin 2017/22**

(73) Proprietor: **Fujifilm Corporation
Minato-ku
Tokyo (JP)**

(72) Inventors:
• **IMAIZUMI, Yuki
Kanagawa (JP)**
• **AIMI, Makiko
Kanagawa (JP)**
• **SUZUKI, Mayuko
Kanagawa (JP)**
• **YANAGI, Terukazu
Kanagawa (JP)**

(74) Representative: **Klunker IP
Patentanwälte PartG mbB
Destouchesstraße 68
80796 München (DE)**

(56) References cited:
**EP-A1- 2 492 333      EP-A1- 2 762 128
WO-A1-2012/172425      JP-A- 2002 087 931
US-A1- 2004 081 633**

**Description**

Technical Field

The present invention relates to an oil-in-water emulsion gel composition, an external preparation for the skin, and a producing method of an oil-in-water emulsion composition.

Background Art

Oil-based components are commonly formulated in external preparations for the skin such as cosmetics with the object of moisturizing the skin. External preparations for the skin, such as milky lotions or creams, formulated with a high content of oil-based components appear white, and have an appearance lacking in transparency. Accordingly, this appearance gives a strong impression of greasiness, and tends to be difficult for people desiring freshness and a transparent feel to accept.

However, in external preparations for the skin having excellent transparency, such as lotions or water-based gels, the solubilization performance of a surfactant is usually used to formulate an oil-based component, making formulation with a high content of the oil-based component difficult. Thus, the moisturizing effect and skin care effect given by oil-based components in external preparations for the skin having excellent transparency generally tends to be lower than milky lotions, creams, and the like that contain a high content of oil-based components.

There is therefore a demand for an emulsion composition that is suited for use as an external preparation for the skin, that is capable of including an oil-based component in an amount at which a sufficient moisturizing effect is obtained from the oil-based component, and that has excellent transparency.

Technology, which employs an oil-based component and an ionic surfactant in a cosmetic having a high content of an oil-based component or a beauty essence, has been proposed as an emulsion cosmetic (for example, see Japanese Patent Application Laid-Open (JP-A) Nos. 2002-087931, 2001-342113).

A ceramide dispersion, serving as an oil-based component, that contains a sucrose fatty acid ester and a nonionic surfactant, and that includes finely dispersed particles under acidic conditions has also been proposed (for example, see JP-A No. 2015-030705).

It has been reported that a emulsion composition that includes a high content of an oil-based component while maintaining transparency has been prepared by using a high pressure emulsifying device to make the oil-based component into a fine emulsion particles. Namely, emulsion particles including a certain content of the oil-based component are made finer to form a nanoemulsion, and as the number of emulsion particles increases, the inter-particle distances decrease, and the particles are closed up. It has been reported that, as a result, the distances between emulsion particles decreases, repulsion between particles orients the particles, and viscosity rises due to the increase in force of repulsion between particles, such that the emulsion composition enters a gel state (for example, see "New Technology and Applications of Dispersion & Emulsion Systems", edited by Kunio Furusawa, pp 808 (2006)).

SUMMARY OF INVENTION

Problem to be Solved

In the technology described by JP-A No. 2002-087931, the emulsion composition is transparent and has excellent emulsion stability, but a gel-state composition is difficult to obtain. In the technology described by JP-A No. 2001-342113, a transparent emulsion composition is obtained, and a gel-state liquid cosmetic is then obtained by adding a thickening agent. However, in the case that the emulsion composition is placed in the gel-state by addition a thickening agent, the thickening agent often causes stickiness.

Although the dispersion described by JP-A No. 2015-030705 is transparent, a gel-state dispersion is difficult to obtain since the dispersion includes a salt.

US 2004/081633 A1 relates to transparent or clear emulsions for cosmetic or pharmaceutical use. The transparent emulsions comprise an oil phase, containing at least one lipophilic solvent; an aqueous phase; and an emulsifying system containing at least one non-ethoxylated fatty acid ester emulsifier having a hydrophilic-lipophilic balance ("HLB") from about 11 to about 16. Preferred non-ethoxylated fatty acid ester emulsifiers are sucrose esters, in particular sucrose palmitate and sucrose laurate.

WO 2012/172425 A1 relates to a stable cosmetic composition having a syrupy texture while not being sticky, and translucent (or even transparent) appearance.

The gel formed with the nanoemulsion described in "New Technology and Applications of Dispersion & Emulsion Systems", edited by Kunio Furusawa, pp 808 (2006) is placed in the gel state by the force of repulsion between emulsion particles, and the gel may therefore collapse as a result of contact with a salt, or unification of the emulsion particles over time.

From viewpoints of the use of external preparations for the skin such as cosmetic products which are used on the skin, the gel described in the publication of Furusawa et al. will easily collapse by being applied to the skin, and therefore feels spreadable, and gives an excellent feeling of use that is not seen in other preparations, since the appearance rapidly changes from a gel to a liquid on the skin.

However, as described above, the gel will sometimes collapse due to small changes such as contact with a salt, and changes to the particle diameters of the emulsion particles, and there is a demand for an emulsion gel composition that has excellent stability over time.

In consideration of the above circumstances, an object of the invention is to provide an oil-in-water emulsion gel composition, an external preparation for the skin, and a producing method of an oil-in-water emulsion composition, having excellent transparency and excellent stability over time.

Means for Solving the Problem

The present invention is defined in the appended claims.

<1> An oil-in-water gel emulsion composition characterized in that it comprises the following component A to component E:

(component A) at least one liquid-state oil selected from the group consisting of a hydrocarbon oil, an aliphatic ester and a silicone oil, wherein a content of the liquid-state oil with respect to a total amount of the oil-in-water gel emulsion composition is 15% by mass or higher;
(component B) at least one non-ionic emulsifying agent having an HLB of 7 or lower selected from the group consisting of a (poly)glyceryl fatty acid and a POE hydrogenated castor oil;
(component C) a sucrose fatty acid ester;
(component D) an ionic surfactant having a glutamic acid as a hydrophilic group which is an acyl glutamic acid salt and/or sodium surfactin; and
(component E) water,

wherein a total content ratio of component B, component C, and component D, with respect to component A, is from 0.1 to 0.5 by mass.

<2> The oil-in-water gel emulsion composition according to <1>, wherein a content ratio of component B, component C, and component D satisfies the following Formula 1 by mass: B:C:D = 1: from 0.2 to 20: from 0.02 to 4 (Formula 1).

<3> The oil-in-water gel emulsion composition according to <1>, wherein a content of component B, component C, and component D, with respect to the total amount of the oil-in-water gel emulsion composition, is from 0.5 % by mass to 10 % by mass, from 0.5 % by mass to 10 % by mass, and from 0.1 % by mass to 2.0 % by mass, respectively.

<4> The oil-in-water gel emulsion composition according to any one of <1> to <3>, wherein component C comprises a sucrose fatty acid ester having an HLB of 3 or higher, and the sucrose fatty acid ester is an ester of sucrose and at least one fatty acid selected from the group consisting of stearic acid, oleic acid, myristic acid, palmitic acid and lauric acid.

<5> The oil-in-water gel emulsion composition according to any one of <1> to <4>, wherein component D comprises acyl glutamic acid salt.

<6> The oil-in-water gel emulsion composition according to any one of <1> to <5>, wherein component D comprises sodium stearoyl glutamate.

<7> The oil-in-water gel emulsion composition according to any one of <1> to <6>, wherein component B comprises a (poly)glyceryl fatty acid having an HLB of 7 or lower.

<8> The oil-in-water gel emulsion composition according to any one of <1> to <7>, further comprising component F, wherein component F is at least one water soluble moisturizer selected from the group consisting of a water soluble alcohol, a sugar and an amino acid.

<9> The oil-in-water gel emulsion composition according to <8>, wherein component F comprises at least one selected from the group consisting of ethanol, glycerin, diglycerin, sucrose, sorbitol, trehalose, POE (10) methylglucoside and trimethylglycine.

<10> The oil-in-water gel emulsion composition according to any one of <1> to <9>, further comprising component G, wherein component G comprises at least one selected from the group consisting of cholesterol, a cholesterol derivative selected among dihydrocholesterol, dehydrocholesterol, cholesteryl oleate, cholesteryl isostearate, cholesteryl hydroxystearate, and POE (5) cholesteryl ethers, a phytosterol, a phytosterol derivative selected among phytosteryl oleate, phytosteryl isostearate, phytosteryl hydroxystearate, and POE (5) phytosteryl ethers, and a higher alcohol selected among cetanol, myristyl alcohol, cetostearyl alcohol, stearyl alcohol, behenyl alcohol, arachyl alcohol, isostearyl alcohol, oleyl alcohol, hexyl decanol, octyl dodecanol, and decyl tetradecanol.

<11> The oil-in-water gel emulsion composition according to <10>, wherein component G comprises at least one selected from the group consisting of cholesterol, a POE (5) cholesteryl ether, a phytosterol, a POE (5) phytosteryl ether, isostearyl alcohol, octyl dodecanol, cetanol and behenyl alcohol.

<12> The oil-in-water gel emulsion composition according to any one of <1> to <11>, wherein component A comprises:

at least one hydrocarbon oil selected from the group consisting of a squalan and a hydrogenated polyisobutene, and

at least one silicone oil selected from the group consisting of a dimethicone, a methyl trimethicone and a cyclopentasiloxane,
wherein a total content of the hydrocarbon oil and the silicone oil with respect to the total amount of the oil-in-water gel emulsion composition is 15 % by mass or higher.

<13> The oil-in-water gel emulsion composition according to any one of <1> to <12>, wherein the oil-in-water gel emulsion composition is produced by a method comprising:

preparing an oil phase composition comprising component A and component B,
preparing an aqueous phase composition comprising component C, component D, and component E, and
emulsifying a mixture of the oil phase composition and the aqueous phase composition under a pressure of 100 MPa or higher.

<14> An external preparation for skin characterized in that it comprises the oil-in-water gel emulsion composition according to any one of <1> to <13>.

<15> A method for producing an oil-in-water gel emulsion composition characterized in that it comprises:

preparing an oil phase composition comprising;

at least one liquid-state oil selected from the group consisting of a hydrocarbon oil, an aliphatic ester and a silicone oil, wherein a content of the liquid-state oil with respect to a total amount of the oil-in-water gel emulsion composition to be produced is 15% by mass or higher, and
at least one non-ionic emulsifying agent having an HLB of 7 or lower selected from the group consisting of a (poly)glyceryl fatty acid and a POE hydrogenated castor oil, and

preparing an aqueous phase composition comprising;

a sucrose fatty acid ester,
an ionic surfactant having a glutamic acid as a hydrophilic group, and
a water, and

emulsifying a mixture of the oil phase composition and the aqueous phase composition under a pressure of 100 MPa or higher.

According to the invention, an oil-in-water emulsion gel composition, an external preparation for the skin, and a producing method of an oil-in-water emulsion composition, having excellent transparency and excellent stability over time are provided.

DESCRIPTION OF EMBODIMENTS

Detailed explanation regarding specific exemplary embodiments of the invention are provided below. However, the invention is not limited to the exemplary embodiments below in any way, and embodiments can be arrived at by making additional modification as appropriate within the scope of the object of the invention.

In the present specification, numerical ranges described using "to" indicate ranges that include both the values before and after the "to" used to describe the numerical range as the minimum value and the maximum value, respectively.

In the present specification, in cases in which there are plural substances corresponding to respective component present in a composition, the content of each component in compositions refers to the total content of the plural corresponding substances present in the composition, unless specifically stated otherwise.

In the present specification, "room temperature" refers to 25°C.

Oil-in-water Emulsion Gel Composition

An oil-in-water emulsion gel composition of the present disclosure includes components A to E, and is an oil-in-water emulsion gel composition in which the ratio of the total content of component B, component C, and component D to component A is from 0.1 to 0.5 by mass (also simply referred to as an emulsion composition hereafter).

A: At least one liquid-state oil selected from a hydrocarbon oil, an aliphatic ester, or a silicone oil, included at a content of 15% by mass or higher with respect to the a total amount of the oil-in-water emulsion composition.
B: At least one non-ionic emulsifying agent having a HLB of 7 or lower, selected from a (poly)glyceryl fatty acid or a POE hydrogenated castor oil.
C: A sucrose fatty acid ester.
D: An ionic surfactant having a glutamic acid as a hydrophilic group which is an acyl glutamic acid salt and/or sodium surfactin.
E: Water.

The "oil-in-water emulsion gel composition" of the present specification refers to an oil-in-water emulsion composition in which emulsion particles included in the oil-in-water emulsion composition are oriented with respect to one another, and in states in which stress is not applied, shape of the emulsion composition is preserved with no fluidity being exhibited at room temperature (25°C).
The oil-in-water emulsion gel composition preferably has a hardness of 10 g or above when measured at room temperature using a hardness tester (rheometer) commonly employed in this field of technology.
The emulsion composition of the present disclosure has excellent transparency and excellent stability over time due to having the configuration described above.
"Transparent" in the present specification refers to the exhibition of a transparent appearance.
Turbidity can be used as an index of transparency. Turbidity can be determined by mesuring the absorbance at 650 nm wavelengths in a 1 cm cell. The transparency of the emulsion composition of the present disclosure, measured using the method described above (for example, measured with an ultraviolet-visible absorption spectrometer manufactured by JASCO corporation) is preferably a turbidity of lower than 0.75, more preferably a turbidity of lower than 0.50, and still more preferably a turbidity of lower than 0.25.
"Excellent stability over time" in the present specification refers to the gel-state being maintained over the long term. More specifically, the hardness of the emulsion composition of the present disclosure is preferably maintained at 10 g or higher over a desired period after preparation.
The emulsion composition of the present disclosure, and each of the components included in the emulsion composition, are explained in detail below.
The emulsion composition of the present disclosure has the form of a gel.
In general, when an emulsion composition has a hardness of 10 g or higher measured using a rheometer, the fluidity of the emulsion composition is decreased, and is in a gel state.
The hardness of the emulsion composition of the present disclosure (also referred to as the gel hardness hereafter) is preferably 10 g or higher, more preferably 25 g orhigher, and still more preferably 40 g or higher.
There are no particular restrictions to the method of measuring the hardness of the emulsion composition, and a known method may be applied.
As the hardness value in the present specification, the hardness of the emulsion composition (gel hardness) employs the maximum load change when measured for a 20 mm diameter disc shaped probe under conditions of a load of 200 g, a speed of 1 mm/sec, and a penetration depth of 20 mm at room temperature (namely, 25°C), using a rheometer (FUDOH manufactured by RHEOTECH, Inc.).

*A: At Least One Liquid-State Oil Selected from a Hydrocarbon Oil, an Aliphatic Ester, or a Silicone Oil, Included with a Content of 15% or Higher by Mass with Respect to a total amound of the Oil-In-Water Emulsion Composition*

The emulsion composition of the present disclosure includes A: at least one liquid-state oil selected from a hydrocarbon oil, an aliphatic ester, or a silicone oil (also referred to as "component A" hereafter), included at a content of 15% or higher by mass with respect to a total amount of the oil-in-water emulsion composition.
In the present specification, "liquid-state oil" refers to an oil-based component that is in a liquid state under an atmosphere at 25°C, and has a solubility in water of 0.1% by mass or lower at 25°C.
The liquid-state oil included in component A is selected from hydrocarbon oils, aliphatic esters, or silicone oils.
A hydrocarbon oil as the liquid-state oil in component A may be derived from an animal, may be derived from a plant, or may be a synthetic product.
Examples of the hydrocarbon oil include squalane, liquid paraffins, hydrogenated polyisobutenes, and cyclohexane. Of these, squalane or the like is preferable from the viewpoint of having an excellent feeling of moisturizing.

Examples of the aliphatic ester include fatty acid esters having from 8 to 22 carbon atoms.

Specific examples of the aliphatic ester include isopropyl myristate, isopropyl palmitate, octyldodecyl myristate, myristyl myristate, butyl myristate, butyl stearate, isostearyl isostearate , diisopropyl sebacate , glyceryl tri(caprylate/caprate), triethyl hexanoin, and triolein. Moreover, the aliphatic ester of the present disclosure also encompasses plant-derived liquid-state oils that include aliphatic acid esters, such as olive oil or jojoba oil.

There are no particular limitations to silicone oils as long as the silicone oil includes a siloxane structure and is a liquid-state silicone oil at room temperature.

Specific examples of the silicone oil include oil-based components that are liquid at room temperature, such as dimethicone, trisiloxane, cyclomethicone, cyclopentasiloxane, methyl trimethicone , caprylyl methicone , phenyl trimethicone , diphenyl dimethicone , or diphenyl siloxy phenyltrimethicone.

Of these, dimethicone, methyl trimethicone, caprylyl methicone, and cyclopentasiloxane are preferable from the viewpoint of having a suitable value for hardness, and from the viewpoint of having an excellent feeling of use without stickiness when the emulsion composition is employed as an external preparation for the skin.

The liquid-state oil as component A may be singly included in the emulsion composition, or two or more kinds thereof may be included in the emulsion composition. In cases in which two or more kinds thereof are included, similar liquid-state oils may be employed, namely, two or more kinds selected from hydrocarbon oils, two or more kinds selected from aliphatic esters, or two or more kinds selected from silicone oils. Moreover, two or more kinds selected from different liquid-state oils, for example, at least one pair of kinds selected from a hydrocarbon oil with an aliphatic ester, a hydrocarbon oil with a silicone oil, or an aliphatic ester with a silicone oil may be employed, or a combination in which at least one kind is respectively selected from each of hydrocarbon oils, aliphatic esters, and silicone oils may be employed.

Of these, from the viewpoint of obtaining a better feeling of use when the emulsion composition of the present disclosure is employed as an external preparation for the skin, at least one liquid-state oil selected from hydrocarbon oils and at least one liquid-state oil selected from silicone oils are preferably employed in combination.

The liquid-state oil as the component A is included at a total content of 15% or higher by mass with respect to the total amount of the emulsion composition.

In the oil-in-water emulsion composition of the present disclosure, emulsion particles including the finely emulsified liquid-state oil are in close proximity to one another and are oriented to form a stable gel. It is therefore preferable to include a liquid-state oil for which emulsion particles can be close proximity to one another to be oriented. The emulsion composition of the present disclosure can form a gel-state having excellent stability over time in which emulsion particles being in close proximity to one another, by including the liquid-state oil at a content of 15% or higher by mass with respect to the total amount of the emulsion composition.

The liquid-state oil serving as component A preferably has a total content of 17% or higher by mass, and more preferably a total content of 19% or higher by mass, with respect to the total amount of the emulsion composition.

From the viewpoint of easily obtaining an emulsion composition having excellent transparency, the content of component A is preferably 40% or lower by mass, and more preferably 30% or lower by mass.

Of these, component A preferably includes a hydrocarbon oil and a silicone oil and has a total content of hydrocarbon oil and silicone oil of 15% or higher by mass with respect to the total amount of the emulsion composition, and component A more preferably includes a hydrocarbon oil and a silicone oil at a total content of 17% or higher by mass with respect to the total amount of the emulsion composition.

More specifically, at least one hydrocarbon oil selected from squalane or hydrogenated polyisobutene, and at least one silicone oil selected from dimethicone, methyl trimethicone, caprylyl methicone or cyclopentasiloxane are preferably included at a total content of 15% or higher by mass, more preferably at a total content of 17% or greater by mass, with respect to the total amount of the emulsion composition.

The content ratio of the hydrocarbon oil to the silicone oil is preferably in a range of from 1:10 to 10:1, and from the viewpoint of improving the feel, is more preferably from 3:1 to 1:3, and still more preferably from 2:1 to 1:2.

*B: At Least One Non-Ionic Emulsifying Agent Having a HLB of 7 or Lower, Selected from a (Poly)glyceryl Fatty Acid or a POE Hydrogenated Castor Oil*

The emulsion composition of the present disclosure includes B: at least one non-ionic emulsifier having a HLB of 7 or lower, selected from a (poly)glyceryl fatty acid or a POE (polyoxyethylene) hydrogenated castor oil (also referred to as "component B" hereafter).

HLB is an index of the balance between hydrophilicity and hydrophobicity commonly employed in the field of surfactants. HLB can be calculated by, for example, using a commonly employed calculation formula, such as the Kawakami's formula expressed by Formula (1) below, or the Oda's formula expressed by Formula (2) below.

$$HLB = 7 + 11.7\log (Mw/Mo) \qquad \text{Formula (1)}$$

In Formula (1), Mw represents the molecular weight of the hydrophilic group, and Mo represents the molecular weight of the hydrophobic group.

$$HLB = (\Sigma \text{ inorganic values} / \Sigma \text{ organic values}) \times 10 \qquad \text{Formula (2)}$$

In Formula (2), inorganic values and organic values are characteristic values allocated to individual compounds described in "organic conceptual diagram". The inorganic values and organic values can be referred to literatures, for example, such as "The Organic Conceptual Diagram, its Fundamentals and Applications", edited by Yoshio Koda (1984).

HLB values listed in a catalogue of commercial products may be employed in cases in which the employed non-ionic emulsifier is a commercial product.

Examples of the (poly)glyceryl fatty acid having a HLB of 7 or lower as component B of the present disclosure, include esters of a monoglycerin or a polyglycerin and a fatty acid having from 8 to 18 carbon atoms. Examples of the fatty acid incllude caprylic acid, capric acid, lauric acid, myristic acid, palmitic acid, stearic acid, oleic acid, or linoleic acid. Examples of the (poly)glyceryl fatty acid having a HLB of 7 or lower include monoglyceryl monomyristate, monoglyceryl monooleate, diglyceryl monooleate, diglyceryl dioleate, tetraglyceryl monooleate, tetraglyceryl dioleate, monoglyceryl monoisostearate, diglyceryl monoisostearate, diglyceryl diisostearate, tetraglyceryl monoisostearate, tetraglyceryl diisostearate, monoglyceryl monostearate, diglyceryl monostearate, diglyceryl distearate, tetraglyceryl monostearate, and tetraglyceryl distearate.

Examples of the POE hydrogenated castor oil having a HLB of 7 or lower include compounds in which a polyoxyethylene (POE) chain(s) is added to a hydrogenated castor oil, and the number of moles of added POE is preferably from 5 to 10.

Examples of the POE hydrogenated castor oil having a HLB of 7 or lower include POE (5) hydrogenated castor oil (HLB = 6) or POE (10) hydrogenated castor oil (HLB = 6.5).

Of these, from the viewpoint of obtaining a more suitable value for the hardness of the emulsion composition, component B is preferably a (poly)glyceryl fatty acid having a HLB of 7 or lower, and more preferably glyceryl stearate (HLB = 3), glyceryl stearate (HLB = 7), glyceryl oleate (HLB = 2.5), glyceryl-4 oleate (HLB = 6), or the like.

The non-ionic emulsifier having a HLB of 7 or lower is included in the emulsion composition of the present disclosure preferably at a content of from 0.5% by mass to 10% by mass, and more preferably at a content of from 1.0% by mass to 6.0% by mass, with respect to the total amount of the emulsion composition.

The emulsion composition of the present disclosure may include a single non-ionic emulsifier having a HLB of 7 or lower, or may include two or more kinds thereof.

*C: Sucrose Fatty Acid Ester*

The emulsion composition of the present disclosure includes C: a sucrose fatty acid ester (also referred to as "component C" hereafter).

Examples of component C include fatty acids having from 12 to 18 carbon atoms, such as sucrose esters of lauric acid, myristic acid, palmitic acid, stearic acid, oleic acid, or linoleic acid.

Specific examples of the sucrose fatty acid ester as component C include sucrose monolaurate, sucrose dilaurate, sucrose trilaurate, sucrose tetralaurate, sucrose monopalmitate, sucrose dipalmitate, sucrose tripalmitate, sucrose tetrapalmitate, sucrose monomyristate, sucrose dimyristate, sucrose trimyristate, sucrose tetramyristate, sucrose monostearate, sucrose distearate, sucrose tristearate, and sucrose tetrastearate.

Of these, a sucrose fatty acid ester having a HLB of 3 or higher in which the fatty acid is at least one selected from stearic acid, oleic acid, myristic acid, palmitic acid, or lauric acid is preferable, a sucrose fatty acid ester having a HLB of 3 or greater in which the fatty acid is at least one selected from oleic acid or stearic acid is particulary preferable, and a sucrose ester of stearic acid having a HLB of from 5 to 15 is still more preferable.

A commercial product may be employed as the sucrose fatty acid ester. There are commercial products, such as sucrose fatty acid monoesters and sucrose fatty acid diesters, which are esterified to different extents and have HLBs that differ according to the composition ratio. Such commercial products are available from Mitsubishi-Kagaku Foods Corporation and Dai-ichi Kogyo Seiyaku Co., Ltd. Such commercial products may be selected in the emulsion composition of the present disclosure in accordance with the object.

The emulsion composition of the present disclosure includes the sucrose fatty acid ester (namely, component C) preferably at a content of from 0.5% by mass to 10% by mass, more preferably at a content of from 1.0% by mass to 5.0% by mass, and still more preferably at a content of from 1.90% by mass to 5.0% by mass, with respect to the total amount of the emulsion composition.

The emulsion composition of the present disclosure may include component C singly, or may include two or more kinds thereof.

*D: An Ionic Surfactant Having a Glutamic Acid as a Hydrophilic Group*

The emulsion composition of the present disclosure includes D: an ionic surfactant having a glutamic acid as a hydrophilic group (also referred to as component D hereafter) which is an acyl glutamic acid salt and/or sodium surfactin.

The hydrophobic group of component D is not particularly limited as long as component D is an ionic surfactant having glutamic acid as a hydrophilic group.

From the viewpoint of increasing the transparency and the gel hardness of the emulsion composition, an acyl glutamic acid salt is preferable.

Examples of the acyl glutamic acid salt include sodium cocoyl glutamate, disodium cocoyl glutamate, potassium cocoyl glutamate, triethanolamine cocoyl glutamate , sodium N-lauroyl-L-glutamate, disodium N-lauroyl-L-glutamate, potassium N-lauroyl-L-glutamate, triethanol amine N-lauroyl-L-glutamate, sodium N-myristoyl-L-glutamate, potassium N-myristoyl-L-glutamate, sodium N-stearoyl-L-glutamate, disodium N-stearoyl-L-glutamate, potassium N-stearoyl-L-glutamate, palm fatty acid sodium glutamate, and lycine sodium dilauroyl glutamate.

Of these, a sodium stearoyl glutamate is preferable from the viewpoint of enabling a more suitable value to be obtained for the hardness of the emulsion composition. Specific examples of the sodium stearoyl glutamate include sodium N-stearoyl-L-glutamate or disodium N-stearoyl-L-glutamate. Potassium N-stearoyl-L-glutamate is also preferably used.

The emulsion composition of the present disclosure may be include component D singly, or may include two or more kinds thereof.

The emulsion composition of the present disclosure includes component D preferably at a content of from 0.1% by mass to 2.0% by mass, more preferably at a content of 0.2% by mass to 1.0% by mass, and still more preferably at a content of 0.4% by mass to 1.0% by mass, with respect to the overall content of the emulsion composition.

*E: Water*

The emulsion composition of the present disclosure includes water from the viewpoint of improving transparency.

As long as the water has excellent compatibility with living organisms, the water employed in the present disclosure is not particularly limited, and purified water, distilled water, ion exchange water, pure water, or ultrapure water, such as MILLI-Q WATER, may be employed therefor.

MILLI-Q WATER refers to ultrapure water obtained using a MILLI-Q WATER purification device, which is an ultrapure water preparing device manufactured by Merck Corporation.

The content of water in the emulsion composition of the present disclosure may be adjusted in accordance with the intended usage of the emulsion composition and the desired feeling of use.

*Content Ratio of Component A, Component B, Component C, and Component D*

The emulsion composition of the present disclosure includes components A to E described above, and the total content ratio of component B, component C, and component D with respect to component A ((B + C + D) / A) is from 0.1 to 0.5 by mass.

The total content ratio of component B, component C, and component D, which contribute to the generation and stabilization of the emulsion particles, with respect to the content of the liquid-state oil, which is component A, is from 0.1 to 0.5 by mass, thereby giving the emulsion composition of the present disclosure excellent transparency and stability over time. Namely, the emulsion composition has excellent transparency due to component A being present as fine emulsion particles, and the emulsion composition has excellent stability over time due to the emulsion particles being stable. Note that the stability over time of the emulsion composition refers to the stability of the gel hardness in the oil-in-water emulsion gel composition.

The total content ratio of component B, component C, and component D to component A ((B +C+ D) / A) is preferably from 0.15 to 0.45 by mass.

The content ratio between component B, component C, and component D included in the emulsion composition of the present disclosure, by mass, preferably satisfies the relationship of Formula1 below.

$$B : C : D = 1 : \text{from } 0.2 \text{ to } 20.0 : \text{from } 0.02 \text{ to } 4 \qquad \text{Formula 1}$$

It is more preferable that the relationship of Formula 2 is satisfied.

$$B : C : D = 1 : \text{from } 0.5 \text{ to } 10 : \text{from } 0.1 \text{ to } 2 \qquad \text{Formula 2}$$

Setting the content ratio of component B, component C, and component D, which contribute to the generation and stabilization of the emulsion particles, within the range described above further improves the stability over time of the obtained emulsion composition.

The emulsion composition of the present disclosure may include other components in addition to components A to E described above, as long as the effects of the invention is exhibited.

Explanation follows regarding other components that may be included in the emulsion composition of the present disclosure.

*F: At Least One Water Soluble Moisturizer Selected from Water Soluble Alcohol, Saccharide, or Amino Acid*

The emulsion composition of the present disclosure may include F: at least one water soluble moisturizer selected from a water soluble alcohol, a saccharide, or an amino acid (also referred to as "component F" hereafter).

Including component F in the emulsion composition of the present disclosure further improves the transparency and gel hardness of the emulsion composition, and also further improves the stability over time of the transparency and the stability over time of the hardness.

Any water soluble alcohol, which is generally employed in external preparations for the skin or the like, may be employed as the water soluble alcohol that may be included in the emulsion composition. Examples of the water soluble alcohol of the present disclosure include ethanol, glycerin, diglycerin, 1,3-butylene glycol, dibutylene glycol, and propylene glycol.

Note that in the present specification, "water soluble alcohol" refers to an alcohol for which, after being added to 25°C water at 1% by mass, agitated, and then being left to stand for one hour, there is no separation that can be seen by eye.

Of these, a polyalcohol that has excellent solubility in water are preferable, such as a polyalcohol with a log P value of lower than 0.2. The log P value is an index that indicates hydrophobicity of organic compounds using a value obtained by taking the common logarithm of the 1-octanol / water partition coefficient. Higher positive values indicate higher degrees of hydrophobicity.

A monosaccharide, a polysaccharide, or any saccharide, which is commonly employed in external preparations for the skin such as cosmetics, may be employed as the saccharide that may be included in the emulsion composition.

Examples of the saccharide of the present disclosure include glucose, sucrose, sorbitol, trehalose, maltose, mannitol, and maltotriose. The saccharide may be a derivative, and, for example, a POE-modified saccharide may be employed therefor. Examples of the POE (polyoxyethylene)-modified saccharide include POE (10) methylglucoside, or POE (20) methylglucoside.

Examples of the amino acid that may be included in the emulsion composition include arginine, alanine, glycine, methionine, aspartic acid, lysine, serine, sarcosine, and trimethylglycine.

Of these, from the viewpoint of further improving the stability of the transparency and the gel hardness, component F is preferably at least one selected from ethanol, glycerin, diglycerin, 1,3-butylene glycol, sucrose, sorbitol, trehalose, POE (10) methylglucoside, or trimethylglycine.

From the viewpoint of obtaining better container compatibility when the emulsion composition of the present disclosure is filled into an airless container and then discharged, component F is preferably at least one selected from glycerin, diglycerin, trehalose, sorbitol, sucrose, POE (10) methylglucoside, or trimethylglycine.

The emulsion composition may include component F singly, or may include two or more kinds thereof.

In cases in which component F is included in the emulsion composition of the present disclosure, the content of component F is preferably from 1% by mass to 50% by mass, more preferably from 2% by mass to 30% by mass, with respect to the total amount of the emulsion composition.

*G: Cholesterol or Cholesterol Derivative, Phytosterol or Phytosterol Derivative, or Higher Alcohol*

The emulsion composition of the present disclosure may include G: at least one selected from cholesterol or a cholesterol derivative, a phytosterol or a phytosterol derivative, or a higher alcohol (also referred to as "component G" hereafter).

Including component G in the emulsion composition of the present disclosure further improves the transparency and gel hardness of the emulsion composition, and also further improves the stability over time of the transparency and the stability over time of the hardness.

The cholesterol and derivative thereof that may be included in the emulsion composition of the present disclosure are cholesterol, dihydrocholesterol, dehydrocholesterol, cholesteryl oleate, cholesteryl isostearate, cholesteryl hydroxystearate, and POE (5) cholesteryl ethers.

The phytosterol and derivative thereof that may be included in the emulsion composition of the present disclosure are phytosterol, phytosteryl oleate, phytosteryl isostearate, phytosteryl hydroxystearate, and POE (5) phytosteryl ethers.

The higher alcohol that may be included in the emulsion composition of the present disclosure is cetanol, myristyl alcohol, cetostearyl alcohol, stearyl alcohol, behenyl alcohol, arachyl alcohol, isostearyl alcohol, oleyl alcohol, hexyl decanol, octyl dodecanol, and decyl tetradecanol.

Of these, component G is preferably cholesterol, a phytosterol, a POE (5) phytosteryl ether, isostearyl alcohol, octyl dodecanol, cetanol, or behenyl alcohol.

The emulsion composition may include component G singly, or may include two or more kinds thereof.

In cases in which component G is included in the emulsion composition of the present disclosure, the content of component G is preferably from 0.1 % by mass to 10% by mass, more preferably from 0.5% by mass to 5% by mass, with respect to the total content of the emulsion composition.

The emulsion composition of the present disclosure, which includes components A to E described above, and which preferably further includes component F, component G, and the like, has fine emulsion particles, and therefore has excellent transparency, maintains gel hardness over long periods of time, and has excellent stability over time.

Moreover, the emulsion composition of the present disclosure is an oil-in-water emulsion composition that includes a high content of liquid-state oil, and therefore has an excellent feeling of use when applied to the skin. The emulsion composition of the present disclosure is therefore well suited to use as an external preparation for the skin such as a cosmetic.

As long as the effect of the present disclosure is exhibited, the emulsion composition of the present disclosure may include components other than the components A to G described above, in addition to including components A to E described above, and component F and component G, which are components preferably employed together therewith.

Various components that can be formulated with ordinary external preparations for the skin such as cosmetics may be employed as the other components in accordance with the object.

Specific examples of the other components include functional components that exhibit useful beautifying effects (such as anti-pimple agents, germicidal disinfectants, lightening agents, keratin softeners, anti-inflammatory agents, and circulation promotors), chelating agents, pH control agents, pH buffering agents, ultraviolet absorbers, fragrances, colorants, organic solvents, and preservatives. Examples of functional components include carotenoids such as astaxanthin, β-carotene, zeaxanthin, lycopene, or lutein.

External Preparation For The Skin

The external preparation for the skin of the present disclosure includes the emulsion composition of the present disclosure described above.

In addition to the emulsion composition of the present disclosure, the external preparation for the skin may include medicinal components required by an external preparation for the skin.

Examples of the external preparation for the skin that the emulsion composition of the present disclosure is particularly well suited to being employed in include cosmetics such as skin care cosmetics (such as lotions and liquid cosmetics), body cosmetics (such as body lotions), and scalp cosmetics. Other than cosmetics, pharmaceuticals for treating skin conditions may be included in the external preparation for the skin; however, there is no limitation to such applications.

A container used for the emulsion composition or the external preparation for the skin that includes the same of the present disclosure is not particularly limited. However, in order to suppress bacteria from entering from the surrounding air, and to enable a sterile state to be achieved over a long usage period, what is known as an airless container (a normal airless container) or a shut-off airless container is preferable. In the present disclosure, emulsion compositions that include component F are preferably used with a shut-off airless container, and shut-off airless containers are more preferably used in cases in which glycerin, trehalose, sorbitol, POE (10) methylglucoside, or trimethyl glycine is included as component F. In cases in which a shut-off airless container is employed, precipitation due to drying arising at a discharge portion, and changes in turbidity, can be suppressed.

Producing Method of Emulsion Composition

The producing method of the emulsion composition of the present disclosure is not particularly limited.

The emulsion composition of the present disclosure, which has excellent transparency and excellent stability over time, is preferably produced by the following producing method.

The producing method of the emulsion composition of the present disclosure includes preparating an oil phase composition including at least one liquid-state oil selected from a hydrocarbon oil, an aliphatic ester, or a silicone oil at a content of 15% or higher by mass, and at least one non-ionic emulsifier having a HLB of 7 or lower selected from a (poly)glyceryl fatty acid ester, or a POE hydrogenated castor oil (an oil phase composition preparation process). The producing method of the emulsion composition of the present disclosure further includes preparing an aqueous phase composition including a sucrose fatty acid ester, an ionic surfactant that has glutamic acid as a hydrophilic group, and water (an aqueous phase composition preparation process). The producing method of the emulsion composition of the present disclosure further includes emulsifying a mixture of the oil phase composition and the aqueous phase composition under a high pressure condition of 100 MPa or higher (an emulsification process).

Oil Phase Composition Preparation Process

In the oil phase composition preparation process, for example, an oil-based component including component A (a liquid-state oil), component B (a non-ionic emulsifier having a HLB of 7 or lower), component G (a cholesterol derivative or a higher alcohol), and the like as desired are mixed together, heated at from 70°C to 120°C, agitated, and caused to dissolve. The oil phase composition is thereby obtained.

Aqueous Phase Composition Preparation Process

In the aqueous phase composition preparation process, for example, a water soluble component including component C (a sucrose fatty acid ester), component D (a ionic surfactant having glutamic acid as a hydrophilic group), component F (a water soluble moisturizer water selected from a soluble alcohol, a saccharide, an amino acid, or an amino acid derivative), and the like as desired is added to water as component E, heated at 70°C, agitated, and caused to dissolve. The aqueous phase composition is thereby obtained.

Emulsifying Process

In the emulsifying process, a mixture is obtained by mixing the aqueous phase composition and the oil phase composition obtained by the above processes, and the obtained mixture is emulsified under a high pressure condition of 100 MPa or higher.

In a case in which an ordinary emulsifying , for example, a device that utilizes the shearing action of a stirrer or impeller agitator, a homo mixer, a continuous flow-type shear device, or the like is employed to perform the emulsification, the shear force is sometimes insufficient, and fine emulsion particles are not obtained during the emulsification,.

The emulsification is therefore preferably performed under a high pressure condition from the viewpoint of the transparency of the obtained emulsion composition and the stability of the gel over time.

The emulsification using a high pressure condition is preferably emulsification that applies a shear force of 100 MPa or higher, and the condition is preferably 150 MPa or higher, and the condition is still more preferably 200 MPa or higher.

Examples of agitation means that can be used to emulsify under a high pressure condition of 100 MPa or higher include high speed agitation methods that employ a homo mixer, a disperser mixer, an ultra mixer, or the like, ultrasound methods that employ an ultrasound homogenizer, and high pressure homogenizer method that apply a high shear force using a high pressure homogenizer.

Examples of ultrasound homogenizers include ultrasound homogenizers US-600, US-1200T, RUS-1200T, MUS-1200T (manufactured by NISSEI Corporation), and ultrasound processors UIP2000, UIP-4000, UIP-8000, and UIP-16000 (manufactured by Hielscher Ultrasonics GmbH). These high output ultrasound irradiation devices are employed at a frequency of 25 kHz or lower, preferably from 15 kHz to 20 kHz.

Examples of chamber-type high pressure homogenizers include a microfluidizer (manufactured by Microfluidics Corporation), a nanomizer (manufactured by Yoshida Kikai Co., Ltd.), and an ultimizer (manufactured by Sugino Machine Limited).

Examples of homogenizing valve-type high pressure homogenizers include Gaulin-type homogenizers (manufactured by APV Co., Ltd.), Rannie-type homogenizers (manufactured by Rannie), high pressure homogenizers (manufactured by Niro Soavi), homogenizers (manufactured by Sanwa Machinery Co., Ltd.), high pressure homogenizers (manufactured by Izumi Food Machinery Co., Ltd.), and ultra high pressure homogenizers (manufactured by IKA Works GmbH & Co. KG).

From the viewpoint of making the emulsion particles fine, the operating pressure of the high pressure homogenizer is preferably 100 MPa or higher, and more preferably 200 MPa or greater.

The number of high pressure treatment passes may be one, and, from the viewpoint of increasing uniformity of the emulsion composition to be obtained, is preferably two or more, and is more preferably from two to five. The temperature of the mixture prior to high pressure dispersion is set to from 20°C to 80°C, but is more preferably from 40°C to 70°C.

The emulsion composition is preferably cooled rapidly to a predetermined temperature using a cooling means immediately after high pressure dispersion. A freely selected commercially available heat exchanger may be employed as the cooling device.

In high pressure emulsifying methods, operating conditions of the emulsifying device, such as the pressure conditions and the temperature conditions described above, may differ according to the device specification, and are not particularly limited.

In the producing method of the present disclosure, the emulsifying process may include a preliminary emulsification. A preliminary emulsified product may be obtained by emulsifying using an ordinary emulsifying device that utilizes the shearing action of a stirrer or impeller agitator, for example, a homo mixer, a continuous flow-type shear device, or the like, under general conditions. Then, the preliminary emulsified product may be further emulsified by high shear processing under a high pressure condition to obtain a transparent emulsion gel composition having the desired fine emulsion particles.

The high shear processing may also be performed repeatedly, if necessary. Moreover, after an emulsified product including the oil phase composition at a higher concentration than in an intended final emulsion composition has been produced, the desired concentration of the emulsion composition may be obtained by diluting with water or an aqueous medium.

Other Processes

In the producing method of the present disclosure, if necessary, a sterilization process may be performed after the emulsion composition has been prepared through each of the processes above.

In cases in which a sterilization process is performed, the sterilization process may be performed during the preparation of the emulsion composition, or may be performed after the preparation of the emulsion composition.

In cases in which the sterilization process is performed during the preparation of the emulsion composition, the sterilization process may be performed at the stage of any of the processes described above. Of those processes, the sterilization process is preferably performed while the mixture is obtained by agitating and mixing the aqueous phase composition with the oil phase composition, or as soon as possible after the emulsification has been performed under high pressure conditions of 100 MPa or greater.

The emulsion composition of the present disclosure, which has excellent transparency and excellent stability over time, is produced by the method described above.

EXAMPLES

Detailed explanation follows regarding examples of the invention. However, the invention is in no way limited to these examples. Note that "%" denotes "% by mass" unless specifically stated otherwise.

*Example 1 to Example 8*

Out of the components listed in Table 1 below, component C, component D, component E, and ethanol serving as component F were heated at 70°C for 45 minutes and dissolved, and an aqueous phase composition A was thereby obtained.

Component A, component B, and tocopherol and astaxanthin-containing oil (extract of haematococcus algae, ASTOTS-S manufactured by Fujifilm Corporation, and astaxanthin at a content of 20%) serving as other components listed in Table 1 below were heated at 70°C for 30 minutes and dissolved, and a oil phase composition A was obtained.

The obtained oil phase composition A was added to the obtained aqueous phase composition A while agitating the aqueous phase composition A, and a preliminary emulsified product was then obtained via one minute of ultrasound treatment per 100 g using an ultrasound homogenizer (model: US-600, NISSEI Corporation).

Next, the obtained preliminary emulsified product was emulsified at a pressure of 245 MPa using an ultra high pressure emulsifying device (model name: Ultimizer HJP-25001, manufactured by Sugino Machine Limited), and an emulsion composition was thereby obtained.

The obtained emulsion composition was evaluated using the method below, and the results are listed in Table 1 below.

*Appearance*

The obtained emulsion composition was inspected by eye, and it was checked whether or not a gel-state was exhibited.

*Hardness Measurement: Initial Hardness*

In order to measure the hardness of the obtained emulsion composition, the stress from a sample when a fixed load was applied was detected as a load, and the detected load served as the hardness (units of g).
A rheometer (FUDOH manufactured by RHEOTECH Inc.) was employed as a hardness measuring device.
As the method of measuring the hardness, when a disc-shaped probe (20Φ) was pushed down 20 mm at a speed of 1 mm/sec with a load of 200 g into the emulsion composition at room temperature, the maximum load value was employed as the hardness, and evaluation were made using the criteria below based on the obtained values of hardness.

Evaluation Criteria

A: Hardness is 40 g or higher
B: Hardness is 25 g or higher but lower than 40 g
C: Hardness is 10 g or higher but lower than 25 g
D: Hardness is lower than 10 g

*Hardness Stability: Changes to Hardness*

The obtained emulsion composition was stored for one month at 50°C, and the hardness of the emulsion composition was then measured similarly to the initial hardness. The difference from the initial hardness was calculated. The smaller the difference is, the better the stability over time is evaluated.

*Hardness Stability: Life Until Hardness of 10 g*

The obtained emulsion composition was stored at 50°C, and the hardness of the emulsion composition was periodically measured in a similar manner to the initial hardness. The measurements continued until the hardness became 10 g, and points in time at which the hardness become 10 g are listed in Table 1 as the storage time (life until hardness of 10 g).
Moreover, evaluations were performed based on the criteria below. The longer the time until the hardness reached 10 g, the better the evaluation for stability over time.

Evaluation Criteria

A: Time is 3 months or greater until hardness became 10 g
B: Time is 2 months or greater, but less than 3 months until hardness became 10 g
C: Time is 1 month or greater, but less than 2 months until hardness became 10 g
D: Time is less than 1 month until hardness became 10 g

*Turbidity Measurement: Initial Turbidity*

A transparency evaluation was made by measuring the absorbance and turbidity at a wavelength of 650 nm using an ultraviolet-visible spectrometer, and the transparency was evaluated based on the criteria below.
The turbidity measurement was performed using a V-730 (manufactured by JASCO Corporation), which is a commercially available ultraviolet-visible spectrometer. Namely, the emulsion composition was placed in a spectroscopic cell and the measurement was performed over a path length of 1 cm, and the absorbance at a wavelength of 650 nm was taken as the turbidity.

Evaluation Criteria

A: Turbidity is less than 0.25
B: Turbidity is 0.25 or greater, but less than 0.50
C: Turbidity is 0.50 or greater, but less than 0.75
D: Turbidity is 0.75 or greater

*Turbidity Measurement: Turbidity Stability*

The obtained emulsion composition was stored at 50°C for 1 month, and the turbidity of the emulsion composition after being stored was measured in a similar manner to the initial turbidity. The difference between the initial turbidity and the turbidity after being stored was calculated.

The turbidity stability was evaluated using the criteria below.

Evaluation Criteria

A: Change in turbidity is less than 0.10
B: Change in turbidity is 0.10 or greater but less than 0.25
C: Change in turbidity is 0.25 or greater but less than 0.50
D: Change in turbidity is 0.50 or greater

Details regarding each component employed in the preparation of the emulsion composition are as follows.

Squalane (component A): purified olive squalane manufactured by Nikko Chemicals Co., Ltd.
Hydrogenated polyisobutene (component A): PARLEAM EX manufactured by NOF corporation
Dimethicone (component A): KF-96A 5cs (viscosity) manufactured by Shin-Etsu Chemical Co., Ltd.
Methyl trimethicone (component A): TMF-1.5 manufactured by Shin-Etsu Chemical Co., Ltd.
Cyclopentasiloxane (component A): KF-995 manufactured by Shin-Etsu Chemical Co., Ltd.
Octyldodecyl myristate (component A): NIKKOL ODM-100 manufactured by Nikko Chemicals Co., Ltd.
Glyceryl tri(caprylate/caprate) (component A): COCONAD MT manufactured by Kao Corporation
Glyceryl stearate, HLB = 3 (component B): NIKKOL MGS BV-2 manufactured by Nikko Chemicals Co., Ltd.
Glyceryl stearate, HLB = 7 (component B): NIKKOL MGS F20V manufactured by Nikko Chemicals Co., Ltd.
Glyceryl oleate, HLB = 2.5 (component B): NIKKOL MGO manufactured by Nikko Chemicals Co., Ltd.
Glyceryl-4 oleate, HLB = 6 (component B): NIKKOL TETRAGLYN 1-OV manufactured by Nikko Chemicals Co., Ltd.
POE (5) hydrogenated castor oil, HLB = 6 (component B): NIKKOL HCO-5 manufactured by Nikko Chemicals Co., Ltd.
Sucrose stearic acid ester, HLB = 3 (component C): SURFHOPE SE COSME C-1803 manufactured by Mitsubishi-Kagaku Foods Corporation
Sucrose stearic acid ester, HLB = 5 (component C): SURFHOPE SE COSME C-1805 manufactured by Mitsubishi-Kagaku Foods Corporation
Sucrose stearic acid ester, HLB = 11 (component C): SURFHOPE SE COSME C-1811 manufactured by Mitsubishi-Kagaku Foods Corporation
Sucrose stearic acid ester, HLB = 15 (component C): SURFHOPE SE COSME C-1815 manufactured by Mitsubishi-Kagaku Foods Corporation
Na stearoyl glutamate (component D): AMISOFT HS-11P manufactured by Ajinomoto Healthy Supply Co., Inc.
Na lysine dilauroylglutamate (component D): PELLICER L-30 manufactured by NOF corporation
Na surfactin (component D): KANEKA SURFACTIN manufactured by Kaneka Corporation
Glycerin (component F): cosmetic grade concentrated glycerin manufactured by Kao Corporation
Trehalose (component F): trehalose manufactured by Hayashibara Co., Ltd.

Table 1

| | | | Example 1 | Example 2 | Example 3 | Example 4 | Example 5 | Example 6 | Example 7 | Example 8 |
|---|---|---|---|---|---|---|---|---|---|---|
| | | | EM-1 | EM-2 | EM-3 | EM-4 | EM-5 | EM-6 | EM-7 | EM-8 |
| Component A | Squalane | Hydrocarbon oil | 19.0% | | 9.50% | 4.75% | 9.50% | 9.50% | 9.50% | 9.50% |
| | Hydrogenated polyisobutene | Hydrocarbon oil | | 19.0% | | | | | | |
| | Dimethicone | Silicone oil | | | 9.50% | 14.25% | | | | |
| | Methyl trimethicone | Silicone oil | | | | | 9.50% | | | |
| | Cyclopentasiloxane | Silicone oil | | | | | | 9.50% | | |
| | Octyldodecyl myristate | Ester oil | | | | | | | 9.50% | |
| | Glyceryl tri(caprylate/caprate) | Ester oil | | | | | | | | 9.50% |
| Component B | Glyceryl stearate | (poly)Glyceryl fatty acid (HLB=3) | 3.10% | 3.10% | 3.10% | 3.10% | 3.10% | 3.10% | 2.10% | 3.10% |
| Component C | Sucrose stearic acid ester | Sucrose fatty acid ester (HLB=11) | 2.90% | 2.90% | 2.90% | 2.90% | 2.90% | 2.90% | 2.90% | 2.90% |
| Component D | Na stearoyl glutamate | Ionic surfactant | 0.40% | 0.40% | 0.40% | 0.40% | 0.40% | 0.40% | 0.40% | 0.40% |
| Component E | Water | | Remainder | Remainder | Remainder | Remainder | Remainder | Remainder | Remainder | Remainder |
| Component F | Ethanol | | 7% | 7% | 7% | 7% | 7% | 7% | 7% | 7% |
| Other components | Tocopherol | | 0.5% | 0.5% | 0.5% | 0.5% | 0.5% | 0.5% | 0.5% | 0.5% |
| | Astaxanthin-containing oil | | 0.0038% | 0.0038% | 0.0038% | 0.0038% | 0.0038% | 0.0038% | 0.0038% | 0.0038% |
| Process | High pressure emulsification | | Yes | Yes | Yes | Yes | Yes | Yes | Yes | Yes |

EP 3 173 064 B1

(continued)

| | | | Example 1 | Example 2 | Example 3 | Example 4 | Example 5 | Example 6 | Example 7 | Example 8 |
|---|---|---|---|---|---|---|---|---|---|---|
| | | | EM-1 | EM-2 | EM-3 | EM-4 | EM-5 | EM-6 | EM-7 | EM-8 |
| Initial hardness | Appearance | | Gel | Gel | Gel | Gel | Gel | Gel | Gel | Gel |
| | Hardness | | 38.3 | 33.6 | 35.2 | 34.0 | 46.5 | 35.9 | 27.4 | 23.6 |
| | Evaluation | | B | B | B | B | A | B | B | C |
| Initial turbidity | Transparency, turbidity | | 0.35 | 0.32 | 0.15 | 0.11 | 0.12 | 0.16 | 0.29 | 0.59 |
| | Evaluation | | B | B | A | A | A | A | B | C |
| Hardness stability | Hardness after one month at 50°C | | 28.7 | 28.7 | 23.2 | 24.0 | 32.1 | 23.6 | 20.2 | 10.1 |
| | Change in hardness after one month at 50°C | | 9.6 | 4.9 | 12.0 | 10.0 | 14.4 | 12.3 | 7.2 | 13.5 |
| | Time until hardness 10 (unit: month) | | 2.9 | 4.8 | 2.1 | 2.4 | 2.5 | 2.1 | 2.4 | 1.0 |
| | Evaluation | | B | A | B | B | B | B | B | C |
| Turbidity stability | Turbidity after one month at 50°C | | 0.65 | 0.62 | 0.27 | 0.17 | 0.24 | 0.24 | 0.51 | 0.64 |
| | Change in turbidity after one month at 50°C | | 0.30 | 0.30 | 0.12 | 0.06 | 0.13 | 0.08 | 0.22 | 0.05 |
| | Evaluation | | C | C | B | A | B | A | B | A |

It is apparent from the results in Table 1 that the emulsion compositions of Example 1 to Example 8 have excellent transparency and are emulsion compositions having sufficient gel hardness for practical use. Of these, it is apparent that the emulsion compositions of Example 3 to Example 6, which include a hydrocarbon oil and a silicone oil as component A, had superior transparency.

*Example 9 to Example 13 and Comparative Example 1*

Emulsion compositions were prepared similarly to Example 1 by following formulations of Table 2 below, and the obtained emulsion compositions were evaluated similarly to Example 1. The results are listed in Table 2 below. For reference, the formulation and evaluation results of Example 3 are also listed in Table 2.

Table 2

| | | Comparative Example 1 | Example 9 | Example 10 | Example 3 | Example 11 | Example 12 | Example 13 |
|---|---|---|---|---|---|---|---|---|
| | | EM-9 | EM-10 | EM-11 | EM-3 | EM-12 | EM-13 | EM-14 |
| Component A | Squalane | 5.00% | 8.00% | 8.50% | 9.50% | 9.50% | 11.2% | 14.3% |
| | Dimethicone | 5.00% | 8.00% | 8.50% | 9.50% | 9.50% | 11.2% | 14.3% |
| | Total content of component A | 10.0% | 16.0% | 17.0% | 19.0% | 19.0% | 22.4% | 28.6% |
| Component B | Glyceryl stearate (HLB=3) | 3.10% | 3.10% | 3.10% | 3.10% | 2.10% | 2.10% | 2.10% |
| Component C | Sucrose stearic acid ester (HLB=11) | 2.90% | 2.90% | 2.90% | 2.90% | 2.90% | 2.90% | 2.90% |
| Component D | Na stearoyl glutamate | 0.40% | 0.40% | 0.40% | 0.40% | 0.40% | 0.40% | 0.40% |
| Component E | Water | Remainder | Remainder | Remainder | Remainder | Remainder | Remainder | Remainder |
| Component F | Ethanol | 7% | 7% | 7% | 7% | 7% | 7% | 7% |
| Other components | Tocopherol | 0.5% | 0.5% | 0.5% | 0.5% | 0.5% | 0.5% | 0.5% |
| | Astaxanthin-containing oil | 0.0038% | 0.0038% | 0.0038% | 0.0038% | 0.0038% | 0.0038% | 0.0038% |
| Process | High pressure emulsification | Yes | Yes | Yes | Yes | Yes | Yes | Yes |
| Initial hardness | Appearance | Liquid | Gel | Gel | Gel | Gel | Gel | Gel |
| | Hardness | 1.9 | 20.1 | 25.4 | 35.2 | 25.1 | 37.5 | 58.1 |
| | Evaluation | D | C | B | B | B | B | A |
| Initial turbidity | Transparency, turbidity | 0.12 | 0.20 | 0.21 | 0.15 | 0.20 | 0.28 | 0.38 |
| | Evaluation | A | A | A | A | A | B | B |
| Hardness stability | Hardness after one month at 50°C | - | 10.0 | 12.1 | 23.2 | 18.8 | 30.6 | 40.2 |
| | Change in hardness after one month at 50°C | - | 10.1 | 13.3 | 12.0 | 6.3 | 6.9 | 17.9 |
| | Life until hardness 10 (unit: month) | - | 1.0 | 1.2 | 2.1 | 2.4 | 4.0 | 2.7 |
| | Evaluation | - | C | C | B | B | A | B |
| Turbidity stability | Turbidity after one month at 50°C | - | 0.36 | 0.25 | 0.27 | 0.40 | 0.38 | 0.43 |
| | Change in turbidity after one month at 50°C | - | 0.16 | 0.04 | 0.12 | 0.20 | 0.10 | 0.05 |
| | Evaluation | - | B | A | B | B | B | A |

EP 3 173 064 B1

18

It is apparent from the results in Table 2 that the emulsion compositions of Example 9 to Example 13, similarly to the emulsion compositions of Example 3, have excellent transparency and are emulsion compositions having sufficient gel hardness for practical use. It is apparent from the evaluation results of Example 3 and Example 9 to Example 13 that the gel hardness tends to increase as the content of component A increases.

On the other hand, the hardness of the obtained emulsion composition of Comparative Example 1, which had a component A content of 15% or lower, was lower than 10 g and exhibited a liquid-state despite being high pressure emulsified, such that a gel-state emulsion composition was not obtained.

*Example 14 to Example 17, and Comparative Example 2 to Comparative Example 6*

Emulsion compositions were prepared similarly to Example 1 by following formulations of Table 3 below, and the obtained emulsion compositions were evaluated similarly to Example 1. The results are listed in Table 3. For reference, the formulation and the evaluation results of Example 3 are also listed in Table 3.

Table 3

| Category | Ingredient | Type | HLB | Example 3 EM-3 | Example 14 EM-15 | Example 15 EM-16 | Example 16 EM-17 | Example 17 EM-18 | Comparative Example 2 EM-19 | Comparative Example 3 EM-20 | Comparative Example 4 EM-21 | Comparative Example 5 EM-22 | Comparative Example 6 EM-23 |
|---|---|---|---|---|---|---|---|---|---|---|---|---|---|
| Component A | Squalane | | | 9.5% | 9.5% | 9.5% | 9.5% | 9.5% | 9.5% | 9.5% | 9.5% | 19.0% | 9.5% |
| | Dimethicone | | | 9.5% | 9.5% | 9.5% | 9.5% | 9.5% | 9.5% | 9.5% | 9.5% | | 9.5% |
| Component B | Glyceryl stearate | (poly)glyceryl fatty acid | 3 | 3.10% | | | | | | 2.10% | | | |
| | Glyceryl stearate | (poly)glyceryl fatty acid | 7 | | 3.10% | | | | | | | | |
| | Glyceryl oleate | (poly)glyceryl fatty acid | 2.5 | | | 3.10% | | | | | | | |
| | Glyceryl-4 oleate | (poly)glyceryl fatty acid | 6 | | | | 3.10% | | | | | | |
| Comparative component | Polyglyceryl-6 isostearate | (poly)glyceryl fatty acid | 10 | | | | | | 3.10% | | | | |
| Component B | POE(5) hydrogenated castor oil | POE hydrogenated castor oil | 6 | | | | | 3.10% | | | | | |
| | Propylene glycol stearate | propylene glycol fatty acid | 3.5 | | | | | | | 3.10% | | | |
| Comparative components | Sorbitan stearate | Sorbitan fatty acid | 5 | | | | | | | | 3.10% | | |
| | Polysorbate-60 | POE sorbitan fatty acid ester | 15 | | | | | | | | | 3.10% | |
| | Beheneth-5 | POE alkylether | 7 | | | | | | | | | | 3.10% |

| | | | HLB | Example 3 | Example 14 | Example 15 | Example 16 | Example 17 | Comparative Example 2 | Comparative Example 3 | Comparative Example 4 | Comparative Example 5 | Comparative Example 6 |
|---|---|---|---|---|---|---|---|---|---|---|---|---|---|
| | | | | EM-3 | EM-15 | EM-16 | EM-17 | EM-18 | EM-19 | EM-20 | EM-21 | EM-22 | EM-23 |
| Component C | Sucrose stearic acid ester | | | 2.90% | 2.90% | 2.90% | 2.90% | 2.90% | 1.90% | 2.90% | 1.90% | 1.90% | 2.90% |
| Component D | Na stearoyl glutamate | | | 0.40% | 0.40% | 0.40% | 0.40% | 0.40% | 0.40% | 0.40% | 0.40% | 0.40% | 0.40% |
| Component E | Water | | | remainder | remainder | remainder | remainder | remainder | remainder | remainder | remainder | remainder | remainder |
| Component F | Ethanol | | | 7% | 7% | 7% | 7% | 7% | 7% | 7% | 7% | 7% | 7% |
| Other components | Tocopherol | | | 0.5% | 0.5% | 0.5% | 0.5% | 0.5% | 0.5% | 0.5% | 0.5% | 0.5% | 0.5% |
| | Astaxanthin-containing oil | | | 0.0038% | 0.0038% | 0.0038% | 0.0038% | 0.0038% | 0.0038% | 0.0038% | 0.0038% | 0.0038% | 0.0038% |
| Process | High pressure emulsification | | | Yes | Yes | Yes | Yes | Yes | Yes | Yes | Yes | Yes | Yes |
| Initial hardness | Appearance | | | Gel | Gel | Gel | Gel | Gel | Liquid | Gel | Liquid | Liquid | Liquid |
| | Hardness | | | 35.2 | 32.5 | 25.4 | 33.4 | 16.2 | 2.3 | 12.8 | 5.4 | 1.7 | 1.7 |
| | Evaluation | | | B | B | B | B | c | D | c | D | D | D |
| Initial turbidity | Transparency, turbidity | | | 0.15 | 0.24 | 0.20 | 0.17 | 0.35 | 0.43 | 0.38 | 0.65 | 3.18 | 0.34 |
| | Evaluation | | | A | A | A | A | B | B | B | C | D | B |
| Hardness stability | Hardness after one month at 50°C | | | 23.3 | 22.3 | 17.2 | 23.6 | 10.2 | - | 8.3 | - | - | - |
| | Chage in hardness after one month at 50°C | | | 11.9 | 10.2 | 8.2 | 9.8 | 6.0 | - | 4.5 | - | - | - |

EP 3 173 064 B1

(continued)

| | | | | Example 3 | Example 14 | Example 15 | Example 16 | Example 17 | Comparative Example 2 | Comparative Example 3 | Comparative Example 4 | Comparative Example 5 | Comparative Example 6 |
|---|---|---|---|---|---|---|---|---|---|---|---|---|---|
| | | | HLB | EM-3 | EM-15 | EM-16 | EM-17 | EM-18 | EM-19 | EM-20 | EM-21 | EM-22 | EM-23 |
| | Time until hardness 10 (unit: month) | | | 2.1 | 2.2 | 1.9 | 2.4 | 1.0 | - | 0.6 | - | - | - |
| | Evaluation | | | B | B | C | B | C | | D | | | |
| Turbidity stability | Turbidity after one month at 50°C | | | 0.27 | 0.37 | 0.28 | 0.28 | 0.46 | | 0.60 | | | |
| | Change in turbidity after one month at 50°C | | | 0.12 | 0.13 | 0.08 | 0.11 | 0.11 | | 0.22 | | | |
| | Evaluation | | | B | B | A | B | B | | B | | | |

It is apparent from the results in Table 3 that the emulsion compositions of Example 14 to Example 17 have excellent transparency, and are emulsion compositions having sufficient gel hardness for practical use, similarly to the emulsion compositions of Example 3. According to the results of Example 3 and Example 14 to Example 17, it is found that superior evaluation results regarding both transparency and gel hardness was obtained by employing a (poly)glyceryl fatty acid as component B.

On the other hand, when a (poly)glyceryl fatty acid having a HLB exceeding 7 (a comparative component) or surfactants outside of the scope of the invention (comparative components) were employed instead of component B, the hardness of the obtained emulsion composition was lower than 10 g, liquid states were exhibited, and a gel-state emulsion composition was not obtained.

*Example 18 to Example 20, Comparative Example 7*

Emulsion compositions were prepared similarly to in Example 1 by following the formulations listed in Table 4 below, and the obtained emulsion compositions were evaluated similarly to Example 1. The results thereof are also listed in Table 4 below. For reference, the formulations of Example 3 and Example 11 and evaluation results thereof are also listed in Table 4.

Table 4

| | | Comparative Example 7 | Example 18 | Example 11 | Example 3 | Example 19 | Example 20 |
|---|---|---|---|---|---|---|---|
| | | EM-24 | EM-25 | EM-12 | EM-3 | EM-26 | EM-27 |
| Component A | Squalane | 9.5% | 9.5% | 9.5% | 9.5% | 9.5% | 9.5% |
| | Dimethicone | 9.5% | 9.5% | 9.5% | 9.5% | 9.5% | 9.5% |
| Component B | Glyceryl stearate (HLB=3) | | 0.50% | 2.10% | 3.10% | 4.40% | 5.70% |
| Component C | Sucrose stearic acid ester (HLB=11) | 2.90% | 2.90% | 2.90% | 2.90% | 2.90% | 2.90% |
| Component D | Na stearoyl glutamate | 0.40% | 0.40% | 0.40% | 0.40% | 0.40% | 0.40% |
| Component E | Water | Remainder | Remainder | Remainder | Remainder | Remainder | Remainder |
| Component F | Ethanol | 7% | 7% | 7% | 7% | 7% | 7% |
| Other components | Tocopherol | 0.5% | 0.5% | 0.5% | 0.5% | 0.5% | 0.5% |
| | Astaxanthin-containing oil | 0.0038% | 0.0038% | 0.0038% | 0.0038% | 0.0038% | 0.0038% |
| Process | High pressure emulsification | Yes | Yes | Yes | Yes | Yes | Yes |
| Initial hardness | Appearance | Liquid | Gel | Gel | Gel | Gel | Gel |
| | Hardness | 9 | 15.1 | 25.1 | 35.2 | 42.1 | 46.5 |
| | Evaluation | D | C | B | B | A | A |
| Initial turbidity | Transparency, turbidity | 0.42 | 0.41 | 0.20 | 0.15 | 0.13 | 0.17 |
| | Evaluation | B | B | A | A | A | A |
| Hardness stability | Hardness after one month at 50°C | - | 10.6 | 18.8 | 23.3 | 35.5 | 39.1 |
| | Change in hardness after one month at 50°C | - | 4.5 | 6.3 | 11.9 | 6.6 | 7.4 |
| | Life until hardness 10 (unit: month) | - | 1.1 | 2.4 | 2.1 | 4.8 | 5.0 |
| | Evaluation | - | C | B | B | A | A |
| Turbidity stability | Turbidity after one month at 50°C | - | 0.52 | 0.40 | 0.27 | 0.16 | 0.15 |
| | Change in turbidity after one month at 50°C | - | 0.10 | 0.20 | 0.12 | 0.03 | -0.02 |
| | Evaluation | - | B | B | B | A | A |

It is apparent from the results in Table 4 that the emulsion compositions of Example 18 to Example 20 have excellent transparency and are emulsion compositions having sufficient gel hardness for practical use. The content of component B is preferably from 0.50% to 1.0% as described above, and as listed in Table 4, advantageous effects tend to be exhibited when the content of component B is 0.50% or higher, and even more advantageous effects tend to be exhibited when the content of component B is in a range of from 1% to 6%.

On the other hand, in Comparative Example 7, which lacks component B, the obtained emulsion composition had a hardness of lower than 10 g and exhibited a liquid state even when high pressure emulsification was performed, and a gel-state emulsion composition was not obtained.

*Example 21 to Example 25, and Comparative Example 8 to Comparative Example 10*

Emulsion compositions were prepared similarly to in Example 1 by following the formulations listed in Table 5 below, and the obtained emulsion compositions were evaluated similarly to Example 1. The results are also listed in Table 5 below. For reference, the formulations of Example 3 and the evaluation results thereof are also listed in Table 5.

Table 5

| Component | | Structure | HLB | Example 21 EM-28 | Example 22 EM-29 | Example 3 EM-3 | Example 23 EM-30 | Example 24 EM-31 | Example 25 EM-32 | Comparative Example 8 EM-33 | Comparative Example 9 EM-34 | Comparative Example 10 EM-35 |
|---|---|---|---|---|---|---|---|---|---|---|---|---|
| Component A | Squalane | | | 9.5% | 9.5% | 9.5% | 9.5% | 9.5% | 9.5% | 9.5% | 9.5% | 9.5% |
| | Dimethicone | | | 9.5% | 9.5% | 9.5% | 9.5% | 9.5% | 9.5% | 9.5% | 9.5% | 9.5% |
| Component B | Glyceryl stearate | (poly)Glyceryl fatty acid | 3 | 3.10% | 3.10% | 3.10% | 3.10% | 3.10% | 3.10% | 3.10% | 3.10% | 3.10% |
| Component C | Sucrose stearic acid ester | Sucrose fatty acid ester | 3 | 2.90% | | | | | | | | |
| | Sucrose stearic acid ester | Sucrose fatty acid ester | 5 | | 2.90% | | | | | | | |
| | Sucrose stearic acid ester | Sucrose fatty acid ester | 11 | | | 2.90% | | | | | | |
| | Sucrose stearic acid ester | Sucrose fatty acid ester | 15 | | | | 2.90% | | | | | |
| | Sucrose oleic acid ester | Sucrose fatty acid ester | 15 | | | | | 2.90% | | | | |
| | Sucrose lauric acid ester | Sucrose fatty acid ester | 15 | | | | | | 2.90% | | | |
| Comparative components | Polyglyceryl-6 isostearate | Polyglyceryl fatty acid ester | 10 | | | | | | | 2.90% | | |
| | Polysorbate-60 | POE sorbitan fatty acid ester | 15 | | | | | | | | 2.90% | |
| | PEG-20 glyceryl isostearate | POE glyceryl fatty acid | 13 | | | | | | | | | 2.90% |
| Component D | Na stearoyl glutamate | | | 0.40% | 0.40% | 0.40% | 0.40% | 0.40% | 0.40% | 0.40% | 0.40% | 0.40% |
| Component E | Water | | | Remainder | Remainder | Remainder | Remainder | Remainder | Remainder | Remainder | Remainder | Remainder |
| Component F | Ethanol | | | 7% | 7% | 7% | 7% | 7% | 7% | 7% | 7% | 7% |

| | | Structure | HLB | Example 21 EM-28 | Example 22 EM-29 | Example 3 EM-3 | Example 23 EM-30 | Example 24 EM-31 | Example 25 EM-32 | Comparative Example 8 EM-33 | Comparative Example 9 EM-34 | Comparative Example 10 EM-35 |
|---|---|---|---|---|---|---|---|---|---|---|---|---|
| Other components | Tocopherol | | | 0.5% | 0.5% | 0.5% | 0.5% | 0.5% | 0.5% | 0.5% | 0.5% | 0.5% |
| | Astaxanthin-containing oil | | | 0.0038% | 0.0038% | 0.0038% | 0.0038% | 0.0038% | 0.0038% | 0.0038% | 0.0038% | 0.0038% |
| Process | High pressure emulsification | | | Yes | Yes | Yes | Yes | Yes | Yes | Yes | Yes | Yes |
| Initial hardness | Appearance | | | Gel | Gel | Gel | Gel | Gel | Gel | Liquid | Liquid | Liquid |
| | Hardness | | | 33.5 | 34.5 | 33.6 | 38.3 | 34.7 | 14.5 | 3.7 | 4.1 | 2.8 |
| | Evaluation | | | B | B | B | B | B | C | D | D | D |
| Initial turbidity | Transparency, turbidity | | | 0.22 | 0.23 | 0.15 | 0.18 | 0.23 | 0.49 | 0.51 | 0.81 | 0.75 |
| | Evaluation | | | A | A | A | A | A | B | C | D | D |
| Hardness stability | Hardness after one month at 50°C | | | 20.1 | 22.6 | 23.3 | 25.4 | 20.4 | 11.5- | - | - | |
| | Change in hardness after one month at 50°C | | | 13.4 | 11.9 | 10.3 | 12.9 | 14.3 | 3.0- | - | - | |
| | Time until hardness 10 (unit: month) | | | 1.8 | 2.1 | 2.3 | 2.2 | 1.7 | 1.5- | - | - | |
| | Evaluation | | | C | B | B | B | C | C | | | |

(continued)

| | | Example 21 | Example 22 | Example 3 | Example 23 | Example 24 | Example 25 | Comparative Example 8 | Comparative Example 9 | Comparative Example 10 |
|---|---|---|---|---|---|---|---|---|---|---|
| Structure | | EM-28 | EM-29 | EM-3 | EM-30 | EM-31 | EM-32 | EM-33 | EM-34 | EM-35 |
| | HLB | | | | | | | | | |
| Turbidity stability | Turbidity after one month at 50°C | 0.31 | 0.43 | 0.27 | 0.25 | 0.26 | 0.55 | | | |
| | Change in turbidity after one month at 50°C | 0.09 | 0.20 | 0.12 | 0.07 | 0.02 | 0.06 | | | |
| | Evaluation | A | B | B | A | A | A | | | |

It is apparent from the results in Table 5 that the emulsion compositions of Example 21 to Example 25 have excellent transparency and are emulsion compositions having sufficient gel hardness for practical use. It is apparent from Table 5 that sucrose fatty acid esters, which serve as component C, are useful in the preparation of emulsion gel compositions having excellent transparency, and of these, sucrose stearic acid ester is better.

On the other hand, in Comparative Example 8 to Comparative Example 10, in which the comparative components were employed instead of component C, the hardness of the obtained emulsion composition was lower than 10 g and liquid states were exhibited even when high pressure emulsification was performed, and a gel-state emulsion composition was not obtained.

*Example 26 to Example 29, and Comparative Example 11*

Emulsion compositions were prepared similarly to in Example 1 by following the formulations listed in Table 6 below, and the obtained emulsion compositions were evaluated similarly to Example 1. The results are also listed in Table 6 below. For reference, the formulations of Example 3 and the evaluation results thereof are also listed in Table 6.

Table 6

| | | Comparative Example 11 | Example 26 | Example 27 | Example 3 | Example 28 | Example 29 |
|---|---|---|---|---|---|---|---|
| | | EM-36 | EM-37 | EM-38 | EM-3 | EM-39 | EM-40 |
| Component A | Squalane | 9.5% | 9.5% | 9.5% | 9.5% | 9.5% | 9.5% |
| | Dimethicone | 9.5% | 9.5% | 9.5% | 9.5% | 9.5% | 9.5% |
| Component B | Glyceryl stearate (HLB=3) | 3.10% | 3.10% | 3.10% | 3.10% | 3.10% | 0.50% |
| Component C | Sucrose stearic acid ester (HLB=11) | | 1.00% | 1.90% | 2.90% | 5.00% | 5.00% |
| Component D | Na stearoyl glutamate | 0.40% | 0.40% | 0.40% | 0.40% | 0.40% | 0.40% |
| Component E | Water | Remainder | Remainder | Remainder | Remainder | Remainder | Remainder |
| Component F | Ethanol | 7% | 7% | 7% | 7% | 7% | 7% |
| Other components | Tocopherol | 0.5% | 0.5% | 0.5% | 0.5% | 0.5% | 0.5% |
| | Astaxanthin-containing oil | 0.0038% | 0.0038% | 0.0038% | 0.0038% | 0.0038% | 0.0038% |
| Process | High pressure emulsification | Yes | Yes | Yes | Yes | Yes | Yes |
| Initial hardness | Appearance | Liquid | Gel | Gel | Gel | Gel | Gel |
| | Hardness | 4.3 | 17.4 | 25.1 | 33.6 | 65.8 | 32.8 |
| | Evaluation | D | C | B | B | A | B |
| Initial turbidity | Transparency, turbidity | 1.05 | 0.35 | 0.24 | 0.15 | 0.15 | 0.18 |
| | Evaluation | D | B | A | A | A | A |
| Hardness stability | Hardness after one month at 50°C | - | 13.0 | 16.0 | 23.3 | 53.7 | 15.0 |
| | Change in hardness after one month at 50°C | - | 4.4 | 9.1 | 10.3 | 12.1 | 17.8 |
| | Time until hardness 10 (unit: month) | - | 1.7 | 1.7 | 2.3 | 4.6 | 1.3 |
| | Evaluation | - | C | C | B | A | C |
| Turbidity stability | Turbidity after one month at 50°C | - | 0.47 | 0.36 | 0.27 | 0.27 | 0.65 |
| | Change in turbidity after one month at 50°C | - | 0.12 | 0.12 | 0.12 | 0.12 | 0.47 |
| | Evaluation | - | B | B | B | B | C |

EP 3 173 064 B1

30

It is apparent from the results in Table 6 that the emulsion compositions of Example 26 to Example 29 have excellent transparency and are emulsion compositions having sufficient gel hardness for practical use. It is apparent from Table 6 that transparency and gel hardness are better when the content of component C is 1.00% or higher, and gel hardness is further increased when the content of component C is 1.90% or higher.

On the other hand, in Comparative Example 11, which lacks component C, the hardness is lower than 10 g and a liquid state is exhibited even when high pressure emulsification is performed, and a gel-state emulsion composition was not obtained.

*Example 30, Example 31, and Comparative Example 12*

Emulsion compositions were prepared similarly to in Example 1 by following the formulations listed in Table 7 below, and the obtained emulsion compositions were evaluated similarly to Example 1. The results are also listed in Table 7 below. For reference, the formulations of Example 3 and the evaluation results thereof are also listed in Table 7.

Table 7

| | | Example 3 | Example 30 | Example 31 | Comparative example 12 |
|---|---|---|---|---|---|
| | | EM-3 | EM-41 | EM-42 | EM-43 |
| Component A | Squalane | 9.5% | 9.5% | 9.5% | 9.5% |
| | Dimethicone | 9.5% | 9.5% | 9.5% | 9.5% |
| Component B | Glyceryl stearate (HLB=3) | 3.10% | 3.10% | 3.10% | 3.10% |
| Component C | Sucrose stearic acid ester (HLB=11) | 2.90% | 2.90% | 2.90% | 2.90% |
| Component D | Na stearoyl glutamate | 0.40% | | | |
| | Na lysine dilauroyl glutamic acid | | 0.50% | | |
| | Na Surfactin | | | 0.50% | |
| | Na alkyl phosphate | | | | 0.50% |
| Component E | Water | Remainder | Remainder | Remainder | Remainder |
| Component F | Ethanol | 7% | 7% | 7% | 7% |
| Other components | Tocopherol | 0.5% | 0.5% | 0.5% | 0.5% |
| | Astaxanthin-containing oil | 0.0038% | 0.0038% | 0.0038% | 0.0038% |
| Process | High pressure emulsification | Yes | Yes | Yes | Yes |
| Initial hardness | Appearance | Gel | Gel | Gel | Liquid |
| | Hardness | 33.6 | 20.3 | 16.6 | 2.3 |
| | Evaluation | B | c | C | D |
| Initial turbidity | Transparency, turbidity | 0.15 | 0.34 | 0.23 | 0.26 |
| | Evaluation | A | B | A | B |
| Hardness stability | Hardness at 50°C after one month | 23.3 | 11.4 | 11.2 | - |
| | Change in hardness at 50°C after one month | 10.3 | 8.9 | 5.4 | - |
| | Time until hardness 10 (unit: month) | 2.3 | 1.2 | 1.2 | - |
| | Evaluation | B | C | C | - |

(continued)

| | | Example 3 | Example 30 | Example 31 | Comparative example 12 |
|---|---|---|---|---|---|
| | | EM-3 | EM-41 | EM-42 | EM-43 |
| Turbidity stability | Turbidity at 50°C after one month | 0.27 | 0.38 | 0.31 | - |
| | Change in turbidity at 50°C after one month | 0.12 | 0.04 | 0.08 | - |
| | Evaluation | B | A | A | - |

It is apparent from the results in Table 7 that the emulsion compositions of Example 30 and Example 31 have excellent transparency and are emulsion compositions having sufficient gel hardness for practical use. It is apparent from Table 7 that including sodium stearoyl glutamate as component D improves gel hardness.

On the other hand, in Comparative Example 12, in which a surfactant that does not contain glutamic acid as the hydrophilic group was employed instead of component D, the obtained emulsion composition had a gel hardness of lower than 10 g and exhibited a liquid state, even when high pressure emulsification was performed, and a gel-state emulsion composition was not obtained.

*Example 32, Example 33, and Comparative Example 13*

Emulsion compositions were prepared similarly to in Example 1 by following the formulations listed in Table 8 below, and the obtained emulsion compositions were evaluated similarly to Example 1. The results are also listed in Table 8 below. For reference, the formulations of Example 3 and the evaluation results thereof are also listed in Table 8.

Table 8

| | | Comparative Example 13 | Example 3 | Example 32 | Example 33 |
|---|---|---|---|---|---|
| | | EM-44 | EM-3 | EM-45 | EM-46 |
| Component A | Squalane | 9.5% | 9.5% | 9.5% | 9.5% |
| | Dimethicone | 9.5% | 9.5% | 9.5% | 9.5% |
| Component B | Glyceryl stearate (HLB=3) | 3.10% | 3.10% | 3.10% | 3.10% |
| Component C | Sucrose stearic acid ester (HLB=11) | 2.90% | 2.90% | 2.90% | 2.90% |
| Component D | Na stearoyl glutamate | | 0.40% | 1.00% | 2.00% |
| Component E | Water | Remainder | Remainder | Remainder | Remainder |
| Component F | Ethanol | 7% | 7% | 7% | 7% |
| Other components | Tocopherol | 0.5% | 0.5% | 0.5% | 0.5% |
| | Astaxanthin-containing oil | 0.0038% | 0.0038% | 0.0038% | 0.0038% |
| Process | High pressure emulsification | Yes | Yes | Yes | Yes |
| Initial hardness | Appearance | Liquid | Gel | Gel | Gel |
| | Hardness | 9.5 | 33.6 | 38.5 | 44.0 |
| | Evaluation | D | B | B | A |
| Initial turbidity | Transparency, turbidity | 0.34 | 0.15 | 0.18 | 0.22 |
| | Evaluation | B | A | A | A |

(continued)

| | | Comparative Example 13 | Example 3 | Example 32 | Example 33 |
|---|---|---|---|---|---|
| | | EM-44 | EM-3 | EM-45 | EM-46 |
| Hardness stability | Hardness after one month at 50°C | - | 23.3 | 30.2 | 24.7 |
| | Change in hardness after one month at 50°C | - | 10.3 | 8.3 | 19.3 |
| | Time until hardness 10 (unit: month) | - | 2.3 | 3.5 | 1.8 |
| | Evaluation | - | B | A | C |
| Turbidity stability | Turbidity after one month at 50°C | - | 0.27 | 0.55 | 0.70 |
| | Change in turbidity after one month at 50°C | - | 0.12 | 0.37 | 0.48 |
| | Evaluation | - | B | C | C |

As described above, it is preferable that the content of sodium stearoyl glutamate as component D be from 0.1% to 2.0%. According to the results in Table 8, in the emulsion compositions of Example 32 and Example 33, when the content of sodium stearoyl glutamate was from 0.40% to 2.00%, the emulsion composition had excellent transparency and sufficient gel hardness for practical use.

On the other hand, in Comparative Example 13, which lacked component D, the obtained emulsion composition had a hardness of lower than 10 g and exhibited a liquid state, even when high pressure emulsification was performed, and a stable emulsion gel composition was not obtained.

*Example 34 to Example 43*

Emulsion compositions were prepared similarly to in Example 1 by following the formulations listed in Table 9 below, and the hardness and transparency of the obtained emulsion compositions were evaluated similarly to Example 1. For reference, the formulations of Example 3 and the evaluation results thereof are also listed in Table 9.

Container compatibility was also evaluated for Example 3, and Example 34 to Example 43, based on the criteria below. The container compatibility was evaluated by inspecting, by eye, the appearance of emulsion compositions discharged from airless containers into which the emulsion compositions were filled. The results are also listed in Table 9 below.

Container compatibility Evaluation

Evaluation samples were produced by filling the obtained emulsion compositions into normal airless containers (SP-200-HVD manufactured by Yoshino Kogyosho Co., Ltd.) and shut-off airless containers (NP-200-HVD manufactured by Yoshino Kogyosho Co., Ltd.) respectively.

The container compatibility evaluation was performed by storing the evaluation samples at 50°C and periodically inspecting, by eye, the appearance of emulsion composition discharged from the airless container, and the following criteria were used for the evaluation.

Evaluation Criteria

A: Occurrence of precipitates was not observed in the emulsion composition, and there were no changes visible by eye observed in the turbidity compared to the initial evaluation sample.
B: Although the occurrence of precipitates in the emulsion composition was not observed, the turbidity was increased compared to the initial evaluation sample.
C: The occurrence of slight precipitates was observed in the emulsion composition.

D: The occurrence of many precipitates was observed in the emulsion composition.

Table 9

| | | | Example 34 | Example 3 | Example 35 | Example 36 | Example 37 | Example 38 | Example 39 | Example 40 | Example 41 | Example 42 | Example 43 |
|---|---|---|---|---|---|---|---|---|---|---|---|---|---|
| | | | EM-47 | EM-3 | EM-48 | EM-49 | EM-50 | EM-51 | EM-52 | EM-53 | EM-54 | EM-55 | EM-56 |
| Component A | Squalane | Hydrocarbon oil | 9.5% | 9.5% | 9.5% | 9.5% | 9.5% | 9.5% | 9.5% | 9.5% | 9.5% | 9.5% | 9.5% |
| | Dimethicone | Silicone oil | 9.5% | 9.5% | 9.5% | 9.5% | 9.5% | 9.5% | 9.5% | 9.5% | 9.5% | 9.5% | 9.5% |
| Component B | Glyceryl stearate (HLB=3) | (poly)Glyceryl fatty acid | 3.10% | 3.10% | 3.10% | 3.10% | 3.10% | 3.10% | 3.10% | 3.10% | 3.10% | 3.10% | 3.10% |
| Component C | Sucrose stearic acid ester (HLB=11) | Sucrose fatty acid ester | 2.90% | 2.90% | 2.90% | 2.90% | 2.90% | 2.90% | 2.90% | 2.90% | 2.90% | 2.90% | 2.90% |
| Component D | Na stearoyl glutamate | Ionic surfactant | 0.40% | 0.40% | 0.40% | 0.40% | 0.40% | 0.40% | 0.40% | 0.40% | 0.40% | 0.40% | 0.40% |
| Component E | Water | | Remainder | Remainder | Remainder | Remainder | Remainder | Remainder | Remainder | Remainder | Remainder | Remainder | Remainder |
| Component F | Ethanol | Monohydric alcohol | | 7% | | | | | | | 7% | 7% | 7% |
| | Glycerin | Polyhydric alcohol | | | 10% | | | | | | 10% | | |
| | Butylene glycol | Polyhydric alcohol | | | | 10% | | | | | | | |
| | Trehalose | Saccharide | | | | | | 10% | | | | 10% | |
| | Sorbitol | Saccharide | | | | | | | 10% | | | | |
| | POE (10) methylglucoside | Saccharide | | | | | | | | 10% | | | |
| | Trimethylglycine | Amino acid | | | | | | | | | 10% | | 10% |
| Other component | Tocopherol | | 0.5% | 0.5% | 0.5% | 0.5% | 0.5% | 0.5% | 0.5% | 0.5% | 0.5% | 0.5% | 0.5% |
| | Astaxanthin-containin g oil | | 0.0038% | 0.0038% | 0.0038% | 0.0038% | 0.0038% | 0.0038% | 0.0038% | 0.0038% | 0.0038% | 0.0038% | 0.0038% |

EP 3 173 064 B1

| | | | Example 34 | Example 3 | Example 35 | Example 36 | Example 37 | Example 38 | Example 39 | Example 40 | Example 41 | Example 42 | Example 43 |
|---|---|---|---|---|---|---|---|---|---|---|---|---|---|
| | | | EM-47 | EM-3 | EM-48 | EM-49 | EM-50 | EM-51 | EM-52 | EM-53 | EM-54 | EM-55 | EM-56 |
| Process | High pressure emulsification | | Yes | Yes | Yes | Yes | Yes | Yes | Yes | Yes | Yes | Yes | Yes |
| Initial hardness | Appearance | | Gel | Gel | Gel | Gel | Gel | Gel | Gel | Gel | Gel | Gel | Gel |
| | Hardness | | 22.1 | 33.6 | 25.2 | 28.2 | 28.5 | 27.7 | 25.9 | 31.7 | 52.7 | 48.0 | 55.4 |
| | Evaluation | | C | B | B | B | B | B | B | B | A | A | A |
| Initial turbidity | Transparency, turbidity | | 0.38 | 0.15 | 0.24 | 0.21 | 0.23 | 0.19 | 0.17 | 0.24 | 0.09 | 0.08 | 0.09 |
| | Evaluation | | B | A | A | A | A | A | A | A | A | A | A |
| Hardness stability | Hardness after one month at 50°C | | 11.4 | 23.3 | 15.0 | 24.4 | 21.3 | 20.7 | 13.2 | 24.8 | 40.9 | 41.4 | 50.0 |
| | Change in hardness after one month at 50°C | | 10.7 | 10.3 | 10.2 | 3.8 | 7.2 | 7.0 | 12.7 | 6.9 | 11.8 | 6.6 | 5.4 |
| | Time until hardness 10 (unit: month) | | 1.1 | 2.3 | 1.5 | 4.8 | 2.6 | 2.5 | 1.3 | 3.1 | 3.6 | 5.7 | 8.5 |
| | Evaluation | | C | B | C | A | B | B | C | A | A | A | A |
| Turbidity stability | Turbidity after one month at 50°C | | 0.50 | 0.25 | 0.30 | 0.27 | 0.28 | 0.29 | 0.25 | 0.24 | 0.16 | 0.11 | 0.11 |
| | Change in turbidity after one month at 50°C | | 0.13 | 0.10 | 0.06 | 0.06 | 0.05 | 0.10 | 0.09 | 0.00 | 0.06 | 0.02 | 0.02 |
| | Evaluation | | B | A | A | A | A | A | A | A | A | A | A |

EP 3 173 064 B1

36

(continued)

| | Example 34 | Example 3 | Example 35 | Example 36 | Example 37 | Example 38 | Example 39 | Example 40 | Example 41 | Example 42 | Example 43 |
|---|---|---|---|---|---|---|---|---|---|---|---|
| | EM-47 | EM-3 | EM-48 | EM-49 | EM-50 | EM-51 | EM-52 | EM-53 | EM-54 | EM-55 | EM-56 |
| Container compatibility | Normal airless container | | | | | | | | | | |
| | C | C | B | C | B | B | B | B | B | B | B |
| | Shut-off airless container | | | | | | | | | | |
| | B | B | A | B | A | A | A | A | A | A | A |

It is apparent from the results in Table 9 that the emulsion compositions of Example 34 to Example 43 have excellent transparency and are emulsion compositions having sufficient gel hardness for practical use.

It is apparent from the results of Table 9 that further including component F tends to improve transparency and gel hardness.

From the viewpoint of container compatibility, none of the emulsion compositions have problems with regards to suitability for airless containers, and of them, emulsion gel compositions including component F are preferably employed with a shut-off airless container. Moreover, it is apparent that emulsion compositions that include at least one selected from glycerin, trehalose, sorbitol, POE (10) methylglucoside, or trimethylglycine as component F and that are filled into a shut-off airless container are preferable. It is apparent that employing the emulsion composition filled into a shut-off airless container can effectively suppress changes in turbidity and precipitation occurring at the discharge portion due to drying.

*Example 44 to Example 54*

Emulsion compositions were prepared similarly to in Example 1 by following the formulations listed in Table 10 below, and the obtained emulsion compositions were evaluated similarly to Example 1. The results are also listed in Table 10 below. For reference, the formulations of Example 3 and the evaluation results thereof are also listed in Table 10.

Table 10

| | | Example 3 | Example 44 | Example 45 | Example 46 | Example 47 | Example 48 | Example 49 | Example 50 | Example 51 | Example 52 | Example 53 | Example 54 |
|---|---|---|---|---|---|---|---|---|---|---|---|---|---|
| | | EM-3 | EM-57 | EM-58 | EM-59 | EM-60 | EM-61 | EM-62 | EM-63 | EM-64 | EM-65 | EM-66 | EM-67 |
| Component A | Squalane | 9.5% | 9.5% | 9.5% | 9.5% | 9.5% | 9.5% | 9.5% | 9.5% | 9.5% | 9.5% | 9.5% | 7.5% |
| | Dimethicone | 9.5% | 9.5% | 9.5% | 9.5% | 9.5% | 9.5% | 9.5% | 9.5% | 9.5% | 9.5% | 9.5% | 7.5% |
| Component B | Glyceryl stearate (HLB=3) | 3.10% | 3.10% | 3.10% | 3.10% | 3.10% | 3.10% | 3.10% | 3.10% | 3.10% | 3.10% | 3.10% | 3.10% |
| Component C | Sucrose stearic acid ester, (HLB=11) | 2.90% | 2.90% | 2.90% | 2.90% | 2.90% | 2.90% | 2.90% | 2.90% | 2.90% | 2.90% | 2.90% | 2.90% |
| Component D | Na stearoyl glutamate | 0.40% | 0.40% | 0.40% | 0.40% | 0.40% | 0.40% | 0.40% | 0.40% | 0.40% | 0.40% | 0.40% | 0.40% |
| Component E | Water | Remainder | Remainder | Remainder | Remainder | Remainder | Remainder | Remainder | Remainder | Remainder | Remainder | Remainder | Remainder |
| Component F | Ethanol | 7% | 7% | 7% | 7% | 7% | 7% | 7% | 7% | 7% | 7% | 7% | 7% |
| Component G | Cholesterol | | 0.50% | | | | 0.50% | 0.55% | 0.55% | 0.55% | 2% | | 2% |
| | BPS-5 | | | 0.50% | | | 0.50% | 0.15% | 0.15% | 0.15% | | 2% | |
| | Isostearyl alcohol | | | | 0.50% | | | | | 0.50% | | | |
| | Octyldodecanol | | | | | 0.50% | | | | | 0.50% | | |
| Other components | Tocopherol | 0.5% | 0.5% | 0.5% | 0.5% | 0.5% | 0.5% | 0.5% | 0.5% | 0.5% | 0.5% | 0.5% | 0.5% |
| | Astaxanthin-containing oil | 0.0038% | 0.0038% | 0.0038% | 0.0038% | 0.0038% | 0.0038% | 0.0038% | 0.0038% | 0.0038% | 0.0038% | 0.0038% | 0.0038% |
| Process | High pressure emulsification | Yes | Yes | Yes | Yes | Yes | Yes | Yes | Yes | Yes | Yes | Yes | Yes |
| Initial hardness | Appearance | Gel | Gel | Gel | Gel | Gel | Gel | Gel | Gel | Gel | Get | Gel | Gel |
| | Hardness | 33.6 | 39.2 | 31.8 | 33.7 | 38.3 | 47.1 | 43.4 | 49.6 | 46.9 | 45.6 | 38.7 | 33.1 |
| | Evaluation | B | B | B | B | B | A | A | A | A | A | B | B |

(continued)

| | | Example 3 | Example 44 | Example 45 | Example 46 | Example 47 | Example 48 | Example 49 | Example 50 | Example 51 | Example 52 | Example 53 | Example 54 |
|---|---|---|---|---|---|---|---|---|---|---|---|---|---|
| | | EM-3 | EM-57 | EM-58 | EM-59 | EM-60 | EM-61 | EM-62 | EM-63 | EM-64 | EM-65 | EM-66 | EM-67 |
| Initial turbidity | Transparency, turbidity | 0.15 | 0.15 | 0.14 | 0.15 | 0.15 | 0.12 | 0.14 | 0.14 | 0.15 | 0.13 | 0.11 | 0.13 |
| | Evaluation | A | A | A | A | A | A | A | A | A | A | A | A |
| Hardness stability | Hardness after one month at 50°C | 23.2 | 32.8 | 23.8 | 27.0 | 29.7 | 38.0 | 35.8 | 41.1 | 37.2 | 34.7 | 27.9 | 27.7 |
| | Change in hardness after one month at 50°C | 10,4 | 6.4 | 8.0 | 6.7 | 8.6 | 9.1 | 7.7 | 8.5 | 9.8 | 11.0 | 10.8 | 5.4 |
| | Time until hardness 10 (unit: month) | 2.3 | 4.6 | 2.7 | 3.5 | 3.3 | 4.1 | 4.3 | 4.6 | 3.8 | 3.3 | 2.7 | 4.3 |
| | Evaluation | B | A | B | A | A | A | A | A | A | A | B | A |
| Turbidity stability | Turbidity after one month at 50°C | 0.27 | 0.28 | 0.18 | 0.26 | 0.22 | 0.18 | 0.18 | 0.19 | 0.19 | 0.16 | 0.12 | 0.15 |
| | Change in turbidity after one month at 50°C | 0.12 | 0.13 | 0.04 | 0.11 | 0.08 | 0.06 | 0.04 | 0.05 | 0.04 | 0.03 | 0.01 | 0.02 |
| | Evaluation | B | B | A | B | A | A | A | A | A | A | A | A |

40

It is apparent from the results in Table 10 that the emulsion compositions of Example 44 to Example 54 have excellent transparency and are emulsion compositions having sufficient gel hardness for practical use. It is apparent from the evaluation results in Table 10 that the stability of hardness over time and the stability of transparency over time tend to improve by containing component G.

*Example 55 to Example 58, and Comparative Example 14*

Emulsion compositions were prepared by following the formulations of the examples listed in Table 11 below.
Preparation of the emulsion compositions of Example 55 to Example 58 was performed in accordance with the high pressure emulsification pressures listed in Table 11, which were varied from 100 MPa to 245 MPa. As listed in Table 11, Example 55 to Example 58 each prepared emulsion compositions with the same formulation, and differed only in the pressure conditions during the high pressure emulsification.
The emulsion composition of Comparative Example 14 was prepared similarly to Example 55 except in that a high pressure emulsification device was not used.
The obtained emulsion compositions were evaluated similarly to Example 1. The results are listed in Table 11 below.

Table 11

| | | Example 55 | Example 56 | Example 57 | Example58 | Comparative Example 14 |
|---|---|---|---|---|---|---|
| | | EM-68 | EM-69 | EM-70 | EM-71 | EM-72 |
| Component A | Squalane | 9.5% | 9.5% | 9.5% | 9.5% | 9.5% |
| | Dimethicone | 9.5% | 9.5% | 9.5% | 9.5% | 9.5% |
| Component B | Glyceryl stearate (HLB=3) | 3.10% | 3.10% | 3.10% | 3.10% | 3.10% |
| Component C | Sucrose stearic acid ester (HLB=11) | 2.90% | 2.90% | 2.90% | 2.90% | 2.90% |
| Component D | Na stearoyl glutamate | 0.40% | 0.40% | 0.40% | 0.40% | 0.40% |
| Component E | Water | Remainder | Remainder | Remainder | Remainder | Remainder |
| Component F | Ethanol | 7.0% | 7.0% | 7.0% | 7.0% | 7.0% |
| | Glycerin | 10.0% | 10.0% | 10.0% | 10.0% | 10.0% |
| Component G | Cholesterol | 0.55% | 0.55% | 0.55% | 0.55% | 0.55% |
| | BPS-5 | 0.15% | 0.15% | 0.15% | 0.15% | 0.15% |
| Other components | Tocopherol | 0.5% | 0.5% | 0.5% | 0.5% | 0.5% |
| | Astaxanthin-containing oil | 0.0038% | 0.0038% | 0.0038% | 0.0038% | 0.0038% |
| Process | High pressure emulsification, | Yes | Yes | Yes | Yes | No |
| | Emulsification pressure / MPa | 245 MPa | 200 MPa | 150 MPa | 100 MPa | - |
| Initial hardness | Appearance | Gel | Gel | Gel | Gel | Liquid |
| | Hardness | 56.9 | 54.8 | 55.9 | 35.0 | 2.4 |
| | Determination | A | A | A | B | D |
| Initial turbidity | Transparency, turbidity | 0.10 | 0.11 | 0.11 | 0.33 | 3.44 |
| | Determination | A | A | A | B | D |

It is apparent from the results of Table 11 that an emulsion gel composition having high transparency to be obtained by performing emulsification at an emulsification pressure of 100 MPa or higher. Moreover, from the viewpoint of production efficiency, the emulsification pressure is preferably 150 MPa or higher, and the emulsification is more preferably performed at a pressure of 200 MPa or higher.

According to the results in Table 11, in Comparative Example 14, in which high pressure emulsification was not performed, the obtained emulsion composition had a hardness of lower than 10 g and exhibited a liquid state, and a gel-state emulsion composition was not obtained.

*Example 59 and Example 60*

Emulsion compositions were prepared similarly to in Example 1, except in that the content of squalane and dimethicone, which served as component A, in the emulsion composition of Example 1 were as listed in Table 12. Feeling of use in cases in which the emulsion compositions were employed as an external preparation for the skin were evaluated based on the criteria below, for the obtained emulsion compositions of Example 59 and Example 60 and for the obtained emulsion compositions of Example 1 and Example 3. The results are listed in Table 12.

*Evaluation as External Preparation For The Skin*

The emulsion compositions of Example 1, Example 3, Example 59, and Example 60 were used as external preparations for the skin by ten subjects according to the method below, and the feeling of use was evaluated.
First, 10 ml (milliliters) of the emulsion composition was applied to the inner side of one forearm and then spread using a finger of the other arm, and the feeling of use was then evaluated based on the criteria below. The most common evaluation rank given by the subjects was taken as the evaluation rank of the evaluation result.

Feeling of use: Feeling of Moisturizing

Evaluation Criteria

    A: Strong feeling of moisturizing
    B: Feeling of moisturizing
    C: No substantial feeling of moisturizing

Feeling of use: Stickiness

Evaluation Criteria

    A: No substantial feeling of stickiness
    B: Feeling of stickiness
    C: Strong feeling of stickiness

Table 12

| | | | Example 1 | Example 3 | Example 59 | Example 60 |
|---|---|---|---|---|---|---|
| | | | EM-1 | EM-3 | EM-73 | EM-74 |
| Component A | Squalane | Hydrocarbon oil | 19.0% | 9.5% | 6.3% | 3.8% |
| | Dimethicone | Silicone oil | | 9.5% | 12.7% | 15.2% |
| Feeling of use | Feeling of moisturizing | | A | B | B | C |
| | Feeling of Stickiness | | C | B | A | A |

It is apparent from the results in Table 12 that using hydrocarbon oil together with silicone oil as component A gives a better feeling of use, especially a feeling of use with regards to stickiness. Of these, it is apparent that Example 3 and Example 59, in which the content ratio by mass of hydrocarbon oil to silicone oil is from 3:1 to 1:3, have further improved feeling of use.

*Example 61*

An emulsion composition having the composition below was prepared similarly to Example 1.

|  | (Total: 100%) |
|---|---|
| *Component* | *Content* (%) |
| squalane | 9.5 |
| dimethicone | 9.5 |
| tocopherol | 0.5 |
| haematococcus algae extract | 0.00375 |
| sucrose stearate | 2.9 |
| glyceryl stearate | 3.1 |
| Na stearoyl glutamate | 0.4 |
| methyl paraoxybenzoate | 0.15 |
| ethanol | 7.0 |
| glycerin | 10.0 |
| cholesterol | 0.55 |
| POE (5) phytosterol | 0.15 |
| (dimethicone/vinyldimethicone) crosspolymer | 0.1 |
| water | Remainder |

*Example 62*

An emulsion composition having the composition below was prepared similarly to Example 1.

|  | (Total: 100%) |
|---|---|
| *Component* | *Content* (%) |
| squalane | 9.5 |
| dimethicone | 9.5 |
| tocopherol | 0.5 |
| haematococcus algae extract | 0.00375 |
| sucrose stearate | 2.9 |
| glyceryl stearate | 3.1 |
| Na stearoyl glutamate | 0.4 |
| methyl paraoxybenzoate | 0.15 |
| ethanol | 7.0 |
| diglycerin | 10.0 |
| cholesterol | 0.55 |
| POE (5) phytosterol | 0.15 |
| isostearyl alcohol | 0.5 |
| (PEG-240/decyltetradeceth-20/HDI) copolymer | 0.1 |
| bisstearyl PEG/PPG-8/6(SMDI/PEG-400) copolymer | 0.1 |
| water | Remainder |

Example 63

An emulsion composition having the composition below was prepared similarly to Example 1.

| | (Total: 100%) |
| Component | Content (%) |
| --- | --- |
| squalane | 9.5 |
| dimethicone | 9.5 |
| tocopherol | 0.5 |
| haematococcus algae extract | 0.00375 |
| sucrose stearate | 2.9 |
| glyceryl stearate | 3.1 |
| Na stearoyl glutamate | 0.4 |
| methyl paraoxybenzoate | 0.15 |
| ethanol | 7.0 |
| sucrose | 10.0 |
| cholesterol | 0.55 |
| POE (5) phytosterol | 0.15 |
| cetanol | 0.5 |
| behenyl alcohol | 0.5 |
| (PEG-150/stearyl alcohol/SMD I) copolymer | 0.1 |
| water | Remainder |

*Example 64*

An emulsion composition having the composition below was prepared similarly to Example 1.

| | (Total: 100%) |
| Component | Content (%) |
| --- | --- |
| squalane | 9.5 |
| dimethicone | 9.5 |
| tocopherol | 0.5 |
| haematococcus algae extract | 0.00375 |
| sucrose stearate | 1.9 |
| glyceryl stearate | 3.1 |
| Na stearoyl glutamate | 0.4 |
| methyl paraoxybenzoate | 0.15 |
| ethanol | 7.0 |
| sucrose | 10.0 |
| cholesterol | 0.55 |
| POE (5) phytosterol | 0.15 |
| decyltetradodecanol | 0.5 |
| (acrylates/vinyl neodeconoate) crosspolymer | 0.1 |
| water | Remainder |

**Claims**

1. An oil-in-water gel emulsion composition **characterized in that** it comprises the following component A to component E:

(component A) at least one liquid-state oil selected from a hydrocarbon oil, an aliphatic ester or a silicone oil, wherein a content of the liquid-state oil with respect to a total amount of the oil-in-water gel emulsion composition is 15% by mass or higher;
(component B) at least one non-ionic emulsifying agent having an HLB of 7 or lower selected from a (poly)glyceryl fatty acid or a POE hydrogenated castor oil;
(component C) a sucrose fatty acid ester;
(component D) an ionic surfactant having a glutamic acid as a hydrophilic group which is an acyl glutamic acid salt and/or sodium surfactin; and
(component E) water,

wherein a total content ratio of component B, component C, and component D, with respect to component A, is from 0.1 to 0.5 by mass.

2. The oil-in-water gel emulsion composition according to claim 1, wherein a content ratio of component B, component C, and component D satisfies the following Formula 1 by mass:

$$B:C:D = 1: \text{from } 0.2 \text{ to } 20: \text{from } 0.02 \text{ to } 4 \text{ (Formula 1)}.$$

3. The oil-in-water gel emulsion composition according to claim 1, wherein a content of component B, component C, and component D, with respect to the total amount of the oil-in-water gel emulsion composition, is from 0.5 % by mass to 10 % by mass, from 0.5 % by mass to 10 % by mass, and from 0.1 % by mass to 2.0 % by mass, respectively.

4. The oil-in-water gel emulsion composition according to any one of claims 1 to 3, wherein component C comprises a sucrose fatty acid ester having an HLB of 3 or higher, and the sucrose fatty acid ester is an ester of sucrose and at least one fatty acid selected from stearic acid, oleic acid, myristic acid, palmitic acid or lauric acid.

5. The oil-in-water gel emulsion composition according to any one of claims 1 to 4, wherein component D comprises an acyl glutamic acid salt.

6. The oil-in-water gel emulsion composition according to any one of claims 1 to 5, wherein component D comprises sodium stearoyl glutamate.

7. The oil-in-water gel emulsion composition according to any one of claims 1 to 6, wherein component B comprises a (poly)glyceryl fatty acid having an HLB of 7 or lower.

8. The oil-in-water gel emulsion composition according to any one of claims 1 to 7, further comprising component F, wherein component F is at least one water soluble moisturizer selected from a water soluble alcohol, a sugar or an amino acid.

9. The oil-in-water gel emulsion composition according to claim 8, wherein component F comprises at least one selected from ethanol, glycerin, diglycerin, sucrose, sorbitol, trehalose, POE (10) methylglucoside or trimethylglycine.

10. The oil-in-water gel emulsion composition according to any one of claims 1 to 9, further comprising component G, wherein component G comprises at least one selected from cholesterol, a cholesterol derivative selected among dihydrocholesterol, dehydrocholesterol, cholesteryl oleate, cholesteryl isostearate, cholesteryl hydroxystearate, and POE (5) cholesteryl ethers, a phytosterol, a phytosterol derivative selected among phytosteryl oleate, phytosteryl isostearate, phytosteryl hydroxystearate, and POE (5) phytosteryl ethers, or a higher alcohol selected among cetanol, myristyl alcohol, cetostearyl alcohol, stearyl alcohol, behenyl alcohol, arachyl alcohol, isostearyl alcohol, oleyl alcohol, hexyl decanol, octyl dodecanol, and decyl tetradecanol.

11. The oil-in-water gel emulsion composition according to claim 10, wherein component G comprises at least one selected from cholesterol, a POE (5) cholesteryl ether, a phytosterol, a POE (5) phytosteryl ether, isostearyl alcohol, octyl dodecanol, cetanol or behenyl alcohol.

12. The oil-in-water gel emulsion composition according to any one of claims 1 to 11, wherein component A comprises:

at least one hydrocarbon oil selected from a squalan or a hydrogenated polyisobutene, and
at least one silicone oil selected from a dimethicone, a methyl trimethicone or a cyclopentasiloxane,
wherein a total content of the hydrocarbon oil and the silicone oil with respect to the total amount of the oil-in-water gel emulsion composition is 15 % by mass or higher.

13. The oil-in-water gel emulsion composition according to any one of claims 1 to 12, wherein the oil-in-water gel emulsion composition is produced by a method comprising:

preparing an oil phase composition comprising component A and component B,
preparing an aqueous phase composition comprising component C, component D, and component E, and
emulsifying a mixture of the oil phase composition and the aqueous phase composition under a pressure of 100 MPa or higher.

14. An external preparation for skin **characterized in that** it comprises the oil-in-water gel emulsion composition according to any one of claims 1 to 13.

15. A method for producing an oil-in-water gel emulsion composition **characterized in that** it comprises:

preparing an oil phase composition comprising;

at least one liquid-state oil selected from a hydrocarbon oil, an aliphatic ester or a silicone oil, wherein a content of the liquid-state oil with respect to a total amount of the oil-in-water gel emulsion composition to be produced is 15% by mass or higher, and
at least one non-ionic emulsifying agent having an HLB of 7 or lower selected from a (poly)glyceryl fatty acid or a POE hydrogenated castor oil, and

preparing an aqueous phase composition comprising;

a sucrose fatty acid ester,
an ionic surfactant having a glutamic acid as a hydrophilic group, and
a water, and

emulsifying a mixture of the oil phase composition and the aqueous phase composition under a pressure of 100 MPa or higher.

**Patentansprüche**

1. Öl-in-Wasser Gel-Emulsionszubereitung **dadurch gekennzeichnet, dass** sie die folgende Komponente A bis Komponente E enthält:

(Komponente A) mindestens ein Öl im flüssigen Zustand, ausgewählt aus einem Kohlenwasserstofföl, einem aliphatischen Ester oder einem Silikonöl, wobei ein Gehalt an dem Öl im flüssigen Zustand, mit Bezug auf eine Gesamtmenge der Öl-in-Wasser Gel-Emulsionszubereitung, 15 Masse% oder mehr beträgt;
(Komponente B) mindestens ein nichtionisches Emulgiermittel mit einem HLB von 7 oder niedriger, ausgewählt aus einer (Poly)glycerylfettsäure oder einem POE hydrierten Rizinusöl;
(Komponente C) einen Saccharosefettsäureester;
(Komponente D) ein ionisches Tensid, das eine Glutaminsäure als eine hydrophile Gruppe aufweist, die ein Acylglutaminsäuresalz und/oder Natriumsurfactin ist; und
(Komponente E) Wasser,

wobei ein Gesamtmengenverhältnis an Komponente B, Komponente C und Komponente D, mit Bezug auf Komponente A, von 0,1 bis 0,5, bezogen auf die Masse beträgt.

2. Öl-in-Wasser Gel-Emulsionszubereitung nach Anspruch 1, wobei ein Mengenverhältnis an Komponente B, Komponente C und Komponente D, bezogen auf die Masse, die folgende Formel 1 erfüllt:

$$B:C:D = 1: von\ 0,2\ bis\ 20: von\ 0,02\ bis\ 4\ (Formel\ 1).$$

3. Öl-in-Wasser Gel-Emulsionszubereitung nach Anspruch 1, wobei ein Gehalt an Komponente B, Komponente C und Komponente D, mit Bezug auf die Gesamtmenge an der Öl-in-Wasser Gel-Emulsionszubereitung, entsprechend von 0,5 Masse% bis 10 Masse%, von 0,5 % Masse% bis 10 Masse% und von 0,1 Masse% bis 2,0 Masse% beträgt.

4. Öl-in-Wasser Gel-Emulsionszubereitung nach einem der Ansprüche 1 bis 3, wobei Komponente C einen Saccharosefettsäureester mit einem HLB von 3 oder höher umfasst, und der Saccharosefettsäureester ein Ester der Saccharose und mindestens einer Fettsäure, ausgewählt aus Stearinsäure, Ölsäure, Myristinsäure, Palmitinsäure oder Laurinsäure ist.

5. Öl-in-Wasser Gel-Emulsionszubereitung nach einem der Ansprüche 1 bis 4, wobei Komponente D ein Acylglutaminsäuresalz umfasst.

6. Öl-in-Wasser Gel-Emulsionszubereitung nach einem der Ansprüche 1 bis 5, wobei Komponente D Natriumstearoylglutamat umfasst.

7. Öl-in-Wasser Gel-Emulsionszubereitung nach einem der Ansprüche 1 bis 6, wobei Komponente B eine (Poly)glycerylfettsäure mit einem HLB von 7 oder weniger umfasst.

8. Öl-in-Wasser Gel-Emulsionszubereitung nach einem der Ansprüche 1 bis 7 ferner enthaltend Komponente F, wobei Komponente F mindestens ein wasserlösliches Befeuchtungsmittel, ausgewählt aus einem wasserlöslichen Alkohol, einem Zucker oder einer Aminosäure ist.

9. Öl-in-Wasser Gel-Emulsionszubereitung nach Anspruch 8, wobei Komponente F mindestens eines ausgewählt aus Ethanol, Glycerin, Diglycerin, Saccharose, Sorbitol, Trehalose, POE (10) methylglucosid oder Trimethylglycin umfasst.

10. Öl-in-Wasser Gel-Emulsionszubereitung nach einem der Ansprüche 1 bis 9, ferner enthaltend Komponente G, wobei Komponente G mindestens eines, ausgewählt aus Cholesterol, einem Cholesterolderivat, ausgewählt aus Dihydrocholesterol, Dehydrocholesterol, Cholesteryloleat, Cholesterylisostearat, Cholesterylhydroxystearat und POE (5) cholesterylethern, einem Phytosterol, einem Phytosterolderivat, ausgewählt aus Phytosteryloleat, Phytosterylisostearat, Phytosterylhydroxystearat, und POE (5) phytosterylethern, oder einem höheren Alkohol, ausgewählt aus Cetanol, Myristylalkohol, Cetostearylalkohol, Stearylalkohol, Behenylalkohol, Arachylalkohol, Isostearylalkohol, Oleylalkohol, Hexyldecanol, Octyldodecanol und Decyltetradecanol, umfasst.

11. Öl-in-Wasser Gel-Emulsionszubereitung nach Anspruch 10, wobei Komponente G mindestens eines ausgewählt aus Cholesterol, einem POE (5) cholesterylether, einem Phytosterol, einem POE (5) phytosterylether, Isostearylalkohol, Octyldodecanol, Cetanol oder Behenylalkohol umfasst.

12. Öl-in-Wasser Gel-Emulsionszubereitung nach einem der Ansprüche 1 bis 11, wobei Komponente A umfasst:

mindestens ein Kohlenwasserstofföl, ausgewählt aus Squalan oder einem hydrierten Polyisobuten, und mindestens ein Silikonöl, ausgewählt aus Dimethicon, einem Methyltrimethicon oder einem Cyclopentasiloxan, wobei ein Gesamtgehalt an dem Kohlenwasserstofföl und dem Silikonöl, mit Bezug auf die Gesamtmenge der Öl-in-Wasser Gel-Emulsionszubereitung, 15 Masse% oder mehr beträgt.

13. Öl-in-Wasser Gel-Emulsionszubereitung nach einem der Ansprüche 1 bis 12, wobei die Öl-in-Wasser Gel-Emulsionszubereitung durch ein Verfahren hergestellt ist, umfassend:

Herstellen einer Ölphasenzubereitung enthaltend Komponente A und Komponente B,
Herstellen einer Wasserphasenzubereitung enthaltend Komponente C, Komponente D und Komponente E, und
Emulgieren der Mischung der Ölphasenzubereitung und der Wasserphasenzubereitung bei einem Druck von 100 MPa oder höher.

14. Externe Zubereitung für die Haut, **dadurch gekennzeichnet, dass** sie die Öl-in-Wasser Gel-Emulsionszubereitung nach einem der Ansprüche 1 bis 13 enthält.

15. Verfahren zur Herstellung einer Öl-in-Wasser Gel-Emulsionszubereitung, **dadurch gekennzeichnet, dass** es umfasst:

Herstellen einer Ölphasenzubereitung enthaltend;

mindestens ein Öl im flüssigen Zustand, ausgewählt aus einem Kohlenwasserstofföl, einem aliphatischen Ester oder einem Silikonöl, wobei ein Gehalt an dem Öl im flüssigen Zustand, mit Bezug auf eine Gesamtmenge der Öl-in-Wasser Gel-Emulsionszubereitung, 15 Masse% oder mehr beträgt; und mindestens ein nichtionisches Emulgiermittel mit einem HLB von 7 oder niedriger, ausgewählt aus einer (Poly)glycerylfettsäure oder einem POE hydrierten Rizinusöl; und

Herstellen einer Wasserphasenzubereitung enthaltend;

einen Saccharosefettsäureester,
ein ionisches Tensid, das eine Glutaminsäure als hydrophile Gruppe aufweist, und
Wasser, und

Emulgieren einer Mischung der Ölphasenzubereitung und der Wasserphasenzubereitung bei einem Druck von 100 MPa oder höher.

**Revendications**

1. Composition d'émulsion en gel huile dans eau, **caractérisée en ce qu'**elle comprend les composant A à Composant E suivants :

   (composant A) au moins une huile à l'état liquide sélectionnée parmi une huile hydrocarbonée, un ester aliphatique, ou une huile de silicone, où une teneur de l'huile à l'état liquide par rapport à une quantité totale de la composition d'émulsion en gel huile dans eau est supérieure ou égale à 15 % en masse ;
   (composant B) au moins un agent émulsifiant non ionique présentant un HLB inférieur ou égal à 7 sélectionné parmi un acide gras de (poly)glycéryle, ou une huile de ricin hydrogénée POE ;
   (composant C) un ester d'acide gras de saccharose ;
   (composant D) un surfactant ionique présentant un acide glutamique comme groupe hydrophile, lequel est un sel acylé d'acide glutamique, et/ou de la surfactine de sodium, et
   (composant E) de l'eau,
   dans laquelle un rapport total des teneurs en composant B, composant C et composant D, par rapport au composant A, s'étend de 0,1 à 0,5 % en masse.

2. Composition d'émulsion en gel huile dans eau selon la revendication 1, dans laquelle un rapport des teneurs en composant B, composant C et composant D satisfait la formule 1 suivante en masse :

$$\text{B} : \text{C} : \text{D} = 1 : \text{de } 0{,}2 \text{ à } 20 : \text{de } 0{,}02 \text{ à } 4 \text{ (formule 1)}.$$

3. Composition d'émulsion en gel huile dans eau selon la revendication 1, dans laquelle une teneur en composant B, composant C et composant D, par rapport à la quantité totale de la composition d'émulsion en gel huile dans eau, s'étend respectivement de 0,5 % en masse à 10 % en masse, de 0,5 % en masse à 10 % en masse, et de 0,1 % en masse à 2,0 % en masse.

4. Composition d'émulsion en gel huile dans eau selon l'une quelconque des revendications 1 à 3, dans laquelle le composant C comprend un ester d'acide gras de saccharose présentant un HLB supérieur ou égal à 3, et l'ester d'acide gras de saccharose est un ester de saccharose, et au moins un acide gras sélectionné parmi l'acide stéarique, l'acide oléique, l'acide myristique, l'acide palmitique, ou l'acide laurique.

5. Composition d'émulsion en gel huile dans eau selon l'une quelconque des revendications 1 à 4, dans laquelle le composant D comprend un sel acylé d'acide glutamique.

6. Composition d'émulsion en gel huile dans eau selon l'une quelconque des revendications 1 à 5, dans laquelle le composant D comprend un stéaroyl-glutamate de sodium.

7. Composition d'émulsion en gel huile dans eau selon l'une quelconque des revendications 1 à 6, dans laquelle le composant B comprend un acide gras de (poly)glycéryle présentant un HLB inférieur ou égal à 7.

8. Composition d'émulsion en gel huile dans eau selon l'une quelconque des revendications 1 à 7, comprenant en outre un composant F, dans laquelle le composant F est au moins un hydratant soluble dans l'eau sélectionné parmi un alcool soluble dans l'eau, un sucre, ou un acide aminé.

9. Composition d'émulsion en gel huile dans eau selon la revendication 8, dans laquelle le composant F comprend au moins un élément sélectionné parmi l'éthanol, la glycérine, la diglycérine, la saccharose, le sorbitol, un tréhalose, un méthylglucoside ou triméthylglycine POE (10).

10. Composition d'émulsion en gel huile dans eau selon l'une quelconque des revendications 1 à 9, comprenant en outre un composant G, dans laquelle le composant G comprend au moins un élément sélectionné parmi un cholestérol, un dérivé de cholestérol sélectionné parmi un dihydrocholestérol, un déhydrocholestérol, un oléate de cholestéryle, un isostéarate de cholestéryle, un hydroxystéarate de cholestéryle, des esters de cholestéryle POE (5), un phytostérol, un dérivé de phytostérol sélectionné parmi un oléate de phytostéryle, un isostéarate de phytostéryle, un hydroxystéarate de phytostéryle, et des esters de phytostéryle POE(5), ou un alcool supérieur sélectionné parmi le cétanol, l'alcool myristylique, l'alcool cétostéarylique, l'alcool stéarylique, l'alcool béhénylique, l'alcool arachylique, l'alcool isostéarylique, l'alcool oléylique, l'hexyldécanol, l'octyldodécanol, et le décyl-tétradécanol.

11. Composition d'émulsion en gel huile dans eau selon la revendication 10, dans laquelle le composant G comprend au moins un élément sélectionné parmi un cholestérol, un ester de cholestéryle POE (5), un phytostérol, un éther de phytostéryle POE (5), l'alcool isostéarylique, l'octyldodécanol, le cétanol, ou l'alcool béhénylique.

12. Composition d'émulsion en gel huile dans eau selon l'une quelconque des revendications 1 à 11, dans laquelle le composant A comprend :

   au moins une huile hydrocarbonée sélectionnée parmi un squalane ou un polyisobutène hydrogéné, et
   au moins une huile de silicone, sélectionnée parmi une diméthicone, une triméthicone de méthyle, ou un cyclopentasiloxane,
   dans laquelle une teneur totale de l'huile hydrocarbonée et de l'huile de silicone par rapport à la quantité totale de la composition d'émulsion en gel huile dans eau est supérieure ou égale à 15 % en masse.

13. Composition d'émulsion en gel huile dans eau selon l'une quelconque des revendications 1 à 12, dans laquelle la composition d'émulsion en gel huile dans eau est produite par un procédé comprenant les étapes consistant à :

   préparer une composition à phase huileuse comprenant le composant A et le composant B ;
   préparer une composition à phase aqueuse comprenant le composant C, le composant D, et le composant E, et
   émulsifier un mélange de la composition à phase huileuse et la composition à phase aqueuse sous une pression supérieure ou égale à 100 MPa.

14. Préparation à usage externe pour la peau, **caractérisée en ce qu'**elle comprend la composition d'émulsion en gel huile dans eau selon l'une quelconque des revendications 1 à 13.

15. Procédé destiné à produire une composition d'émulsion en gel huile dans eau, **caractérisé en ce qu'**il comprend les étapes consistant à :

   préparer une composition à phase huileuse comprenant :
   au moins une huile à l'état liquide sélectionnée parmi une huile hydrocarbonée, un ester aliphatique, ou une huile de silicone, où une teneur de l'huile à l'état liquide par rapport à une quantité totale de la composition d'émulsion en gel huile dans eau est supérieure ou égale à 15 % en masse ;
   au moins un agent émulsifiant non ionique présentant un HLB inférieur ou égal à 7 sélectionné parmi un acide gras de (poly)glycéryle, ou une huile de ricin hydrogénée POE, et
   préparer une composition à phase aqueuse comprenant :

      un ester d'acide gras de saccharose ;
      un surfactant ionique présentant un acide glutamique comme groupe hydrophile, et
      de l'eau, et

   émulsifier un mélange de la composition à phase huileuse et de la composition à phase aqueuse sous une pression supérieure ou égale à 100 MPa.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002087931 A
- JP 2001342113 A
- JP 2015030705 A
- US 2004081633 A1
- WO 2012172425 A1

**Non-patent literature cited in the description**

- New Technology and Applications of Dispersion & Emulsion Systems. 2006, 808
- The Organic Conceptual Diagram, its Fundamentals and Applications. 1984